# EUROPEAN PATENT APPLICATION

(11) **EP 4 524 151 A1**
(43) Date of publication of application: **19.03.2025**
(21) Application number: 23803837.6
(22) Date of filing: 10.05.2023
(51) Int. Cl.: C07K 14/54, A61K 38/20, A61P 35/00

(54) **INTERLEUKIN21 VARIANT AND FUSION PROTEIN COMPRISING THE SAME AND USES THEREOF**

(30) Priority: 10.05.2022 KR 20220057356
(71) Applicant: Ajou University Industry-Academic Cooperation Foundation, Suwon-si, Gyeonggi-do 16499 (KR); Korea University Research and Business Foundation, Seoul 02841 (KR)
(72) Inventor: KIM, Yong Sung, Suwon-si Gyeonggi-do 16504 (KR); KIM, Eunji, Gwangyang-si Jeollanam-do 57761 (KR); KIM, Jun-Ho, Hwaseong-si Gyeonggi-do 18429 (KR); LEE, Kyung-Mi, Seoul 06001 (KR); YU, Young-Bin, Seoul 02434 (KR)
(74) Representative: Mewburn Ellis LLP
(86) International application number: PCT/KR2023/006343
(87) International publication number: WO 2023/219414

(57) **Abstract**

The present invention relates to an interleukin-21 variant, a fusion protein comprising the interleukin-21 variant and an antibody heavy chain constant region, and uses thereof, and relates to: an interleukin-21 variant in which some amino acids in wild-type interleukin-21 are substituted and/or deleted; a fusion protein in which the interleukin-21 variant is fused with an antibody (immunoglobulin) heavy chain constant region (Fc) pair comprising a first Fc region and a second Fc region; use of the interleukin-21 variant or the fusion protein for preventing or treating cancer; and a method for culturing immune cells using the interleukin-21 variant or the fusion protein.

## Description

### [Technical Field]

The present invention relates to an interleukin 21 variant, a fusion protein containing the interleukin 21 variant and an antibody heavy chain constant region, and uses thereof, and more particularly to an interleukin 21 variant in which one or more amino acids are substituted and/or deleted from wild-type interleukin 21, a fusion protein in which an antibody (immunoglobulin) heavy chain constant region (Fc) pair including a first Fc region and a second Fc region is fused to the interleukin 21 variant, uses of the interleukin 21 variant or fusion protein for preventing or treating cancer, and a method of culturing immune cells using the interleukin 21 variant or fusion protein.

### [Background Art]

Cytokines are relatively small immune proteins contained in the blood. Cytokines secreted from the cells affect other cells or the cells secreting the cytokines, thus playing an important role in cell signaling.

Cytokines are proteins essential for bioregulation, inducing cell proliferation and differentiation, and promoting changes in function and activity, and are related to immune system diseases, allergies, atopy, inflammation, and various types of tumors. Recently, along with the development of immunotherapy and immune cell therapy, the demand for research and medical cytokine proteins used in basic experiments for immune cell culture and cell therapy development is increasing exponentially.

Interleukin-21 (IL21) is a cytokine. IL21 is a member of the IL2 family of cytokines, which includes IL4, IL7, IL9, IL13, and IL15. Proteins in this family have been shown to have both anticancer and antiviral effects.

IL21 is produced by activated helper T (CD4⁺ helper T cells, TH1) cells and natural killer T (NK-T) cells, and is known as an essential regulator of immunity. In particular, IL21 activates the proliferation and cytotoxicity effector functions of CD8+ cytotoxic T lymphocytes and natural killer (NK) cells, two lymphocyte classes that eradicate tumor- and virus-infected cells (Spolski et al., Nat Rev Drug Discov. 2014;13(5):379-95). In addition, IL21 stimulates a selected class of B cells. Therefore, IL21 is essential for immunotherapy as a cytokine that induces the proliferation and activation of immune cells, especially NK cells. However, IL-2 is the only cytokine that has been approved for clinical use for direct administration to patients to date. IL2 increases the survival and activity of NK cells and is relatively inexpensive, but has the disadvantage of suppressing anticancer immune function by activating regulatory T cells, which are immunosuppressive cells. Therefore, there is an urgent need for commercialization of cytokines that activate NK cells without activating regulatory T cells.

In this regard, IL21 has recently been in the spotlight in anticancer immunotherapy. IL21, similar to IL2, promotes the proliferation and differentiation of CD4-positive T cells and CD8-positive T cells. On the other hand, IL21 has been reported to not affect the proliferation of regulatory T cells, and thus its combined administration with anticancer immune cell therapy is anticipated (Al-Chami et al., Cytokine, 2016;82:33-37). In addition, IL21 promotes the differentiation of CD8 T cells into memory cells (Li et al., Journal of Immunology, 2005), and in particular, a recent study reported that IL21 induces genetic reprogramming of differentiated T cells when administered along with HDAC inhibitors and acquires stable memory-associated transcriptional signatures (increased Lef1 and Tcf7), enabling dedifferentiation into memory cells (Wang et al., Cancer Immunology Research, 2020). IL21 has been reported to have the following effects on NK cells: 1) when FMS-like tyrosine kinase-3 (FLT3-L) is present, IL21 along with IL15 facilitates the proliferation of NK cells derived from human CD34-positive hematopoietic progenitor cells (Parrish-Novak et al. Nature, 2000;408(6808):57-63), 2) when membrane-bound form IL21 is expressed in K562 cells used as a nutrient cell line, it facilitates the *in vitro* expansion of NK cells and suppresses aging (Lee DA et al. PlosOne, 2012;7(1):e30264), 3) proliferation by IL2 and IL15 was facilitated when canine NK cells are cultured (Shin et al., Vet Immunol Immunopathol. 2015;165(1):1-13), 4) facilitates the expression of NKG2D receptors by activating STAT3 in human NK cells (Zhu et al., Blood, 2014;124(3):403-411) and the like.

Recombinant human IL21 exhibited antitumor activity in preclinical and clinical trials. However, when administered systemically, IL21 had a short half-life and was consumed by other immune cells expressing the IL21 receptor, which had difficulty in targeting the tumor site and was inevitably administered at a high concentration. In addition, phase 1 clinical trials of IL21 failed due to toxicity resulting from pleiotropic function that simultaneously acts on various immune cells (Petrella et al., Journal of Clinical Oncology, 2013). Several attempts have been made to overcome these shortcomings. In an attempt to increase the half-life, a mono-IL21-Fc fusion protein in which IL21 is bound to the heterodimeric heavy chain constant region (heterodimeric Fc) of an antibody in a monomeric form (Korean Patent No. 10-1928981; U.S. Patent No. US 10800825B2) was suggested. In addition, there was an attempt to develop an immunocytokine (antibody-cytokine conjugate) in which IL21 is fused to an antibody that targets tumors (Shen et al. Front. Immunol. 2020;11:832). However, the protein structure of IL21 has many positively charged amino acids exposed on the surface, and has a problem of reduced stability compared to IL2 and IL4. The problems are that, when administered systemically, IL21 is distributed to other cells and tissues in the body and is readily decomposed, leading to a short serum half-life, ultimately resulting in poor pharmacokinetics, and being undesirable as therapeutic agents. To solve these problems, attempts have been made to introduce mutations capable of reducing positive charges in the recombinant IL21 and to impart other structural stability (US Patent No. US8,475,784B2; Bondensgaard et al. J. BioI. Chem. 2007;282(32):23326-23336). Nevertheless, the development of mutants that can improve the physical properties of IL21 and provide a long half-life is still required.

Human antibodies (Immunoglobulin G (IgG), IgM, IgD, IgE, IgA) that exist in nature are present in the form of an assembly of two heavy chains with the same amino acid sequence and two light chains with the same sequence. In this case, homodimerization between the two identical heavy chains is induced by a non-covalent interaction between the last domains (CH3 domain for IgG, IgD, and IgA, CH4 domain for IgM, and CH2 and CH4 domains for IgE) of the constant region of the antibody, and a disulfide bond between the hinge regions.

The antibody-derived heterodimeric heavy chain constant region (heterodimeric Fc) is a technology that forms a heterodimeric heavy chain constant region by engineering to impart a bond that favors heterodimerization and disfavors or rejects homodimerization through a specific non-covalent bond to the interaction surface between the last domains of the constant region that greatly contributes to the homodimerization of previously naturally occurring antibodies (IgG, IgM, IgA, IgD, and IgE). More specifically, the heterodimeric Fc technology induces mutations in the CH3 domains of two different antibody heavy chains through genetic engineering to form a heterodimer having two heavy chains, similar to a naturally derived antibody, and a minimal deviation in sequence. The EW-RVT heterodimeric Fc mutant used herein is, as a novel strategical mutant, a mutant that promotes the formation of a heterodimer by forming a selective electrostatic bond at the CH3 domain interaction surface (K360E CH3A-Q347R CH3B) and forming a complementary hydrophobic bond instead of the conventional electrostatic bond (K409W CH3A-D399V CH3B/F405T CH3B) through sequence and structural analysis of the interactions of conventional mutants (US Patent No. 9,951,145; Korea Patent No. 1,522,954; Choi HJ et al. Molecular Cancer Therapeutics, 2013;12(12): 2748-2759). The conventional mono-IL21-Fc fusion protein was produced based on EW-RVT heterodimeric Fc (y1/2/4, EW-RVT) in which the sequence of human IgG1, IgG2, and IgG4 is introduced in the hinge-CH2-CH3 region (Korean Patent No. 10-1928981; U.S. Patent No. US 10800825 B2). The mono-IL21-Fc fusion protein thus produced can exhibit superior NK cell proliferation promotion function compared to bi-IL21-Fc in which IL21 was fused to wild-type Fc (y1/2/4), and can induce a superior antitumor effect compared to soluble IL21 or Bi-IL21-Fc by administration in a reasonable frequency to the body (Korean Patent No. 10-1928981; U.S. Patent No. US10800825B2).

The result of the molecular structure analysis of IL21 showed that the amino acids with positively charged side chains were relatively abundant compared to cytokines (IL-2, IL-12, IL-15, IL-18, IFNs) that affect regulation of the proliferation and activation of CD8⁺ cytotoxic T lymphocytes and natural killer (NK) cells, and eventually, IL21 has an isoelectric point (pI) of 9.42 and thus is positively charged under body pH conditions. In addition, it was predicted that the ionic patch formed by positively charged amino acids on the surface of the tertiary structure could interact nonspecifically with various receptors and cells in the body, thus resulting in adverse effects when administered to the body.

Under these technical backgrounds and limitations, the present inventors developed an interleukin 21 variant in which one or more amino acids are substituted and/or deleted in wild-type interleukin 21, and a fusion protein which is a heterodimer containing the interleukin 21 variant, and an antibody (immunoglobulin) heavy chain constant region (Fc) pair including a first Fc region and a second Fc region, wherein the CH3 domain of the first Fc region or the second Fc region has a mutation. Based on this, the present invention has been completed. The mutant developed according to the present invention was found to have superior biological activity and antitumor activity compared to the conventional mono-IL21-Fc fusion protein.

### [Disclosure]

Therefore, the present invention has been made in view of the above problems, and it is one object of the present invention to provide an interleukin 21 variant in which one or more amino acid sequences are substituted or deleted from wild-type interleukin 21.

It is another object of the present invention to provide a fusion protein containing the interleukin 21 variant and an antibody (immunoglobulin) heavy chain constant region (Fc) pair.

It is another object of the present invention to provide a nucleic acid encoding the interleukin 21 variant or fusion protein.

It is another object of the present invention to provide an expression vector containing the nucleic acid.

It is another object of the present invention to provide a transformed recombinant cell containing the expression vector.

It is another object of the present invention to provide a method of producing the interleukin 21 variant or fusion protein.

It is another object of the present invention to provide a composition for preventing or treating cancer containing the interleukin 21 variant or fusion protein as an active ingredient. It is another object of the present invention to provide a method of preventing or treating cancer containing the interleukin 21 variant or fusion protein as an active ingredient. It is another object of the present invention to provide the use of the interleukin 21 variant or fusion protein for the preparation of a composition for preventing or treating cancer.

It is another object of the present invention to provide a method of preventing or treating cancer including administering the interleukin 21 variant or fusion protein to a patient.

It is another object of the present invention to provide a culture method including treating immune cells with the interleukin 21 variant or fusion protein.

In accordance with one aspect of the present invention, the above and other objects can be accomplished by the provision of an interleukin 21 (IL21) variant in which one or more selected from the group consisting of amino acid sequences at positions 50, 54, 75 to 88 and 124 from an N-terminus in an interleukin 21 (IL21) including an amino acid sequence of SEQ ID NO. 4 are substituted or deleted.

In accordance with another aspect of the present invention, provided is a fusion protein, as a heterodimer, containing the interleukin 21 variant and an antibody (immunoglobulin) heavy chain constant region (Fc) pair including a first Fc region and a second Fc region, wherein the Fc pair has a mutation in a CH3 domain of the first Fc region or the second Fc region.

In accordance with another aspect of the present invention, provided is a nucleic acid encoding the interleukin 21 variant or the fusion protein.

In accordance with another aspect of the present invention, provided is an expression vector containing the nucleic acid.

In accordance with another aspect of the present invention, provided is a transformed recombinant cell containing the expression vector.

In accordance with another aspect of the present invention, provided is a method of producing an interleukin 21 variant or fusion protein including:

(a) culturing the cell; and (b) harvesting the interleukin 21 variant or fusion protein from the cultured cell.

In accordance with another aspect of the present invention, provided is a composition for preventing or treating cancer containing the interleukin 21 variant or fusion protein as an active ingredient. In accordance with another aspect of the present invention, provided is a method for preventing or treating cancer including administering the interleukin 21 variant or fusion protein to a patient. In accordance with another aspect of the present invention, provided is the use of the interleukin 21 variant or fusion protein for the preparation of a drug for preventing or treating cancer.

In accordance with another aspect of the present invention, provided is a culture method including treating an immune cell with the interleukin 21 variant or fusion protein.

### [Description of Drawings]

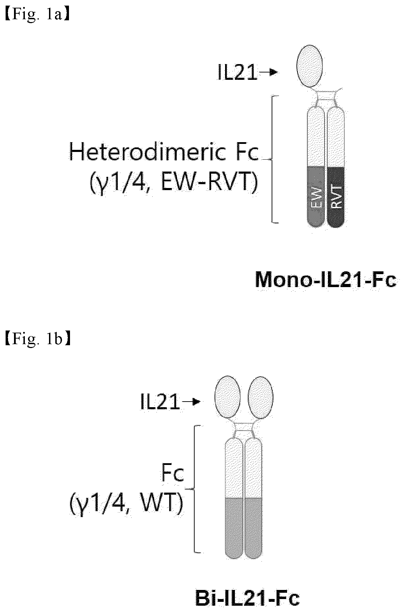

FIG. 1A is a schematic diagram illustrating a mono-IL21-Fc fusion protein (IL21 monomer-Fc fusion protein) constructed by introducing human IL21 cytokine in the form of a monovalent monomer into EW-RVT heterodimeric Fc. The EW-RVT heterodimeric Fc used herein is an Fc (y1/4) that has the hinge region sequence of human IgG1 and the CH2-CH3 region of human IgG4, into which an EW-RVT mutation is introduced. "Mono" means a monomer.

FIG. 1B is a schematic diagram illustrating a Bi-IL21-Fc fusion protein (IL21 dimer-Fc fusion protein) constructed by introducing human IL21 cytokine in the form of a bivalent dimer into wild-type Fc, as a comparative example of FIG. 1A. The wild-type Fc used herein is an Fc (γ1/4) that has the hinge region sequence of human IgG1 and the CH2-CH3 region of human IgG4. "Bi" means a bivalent dimer.

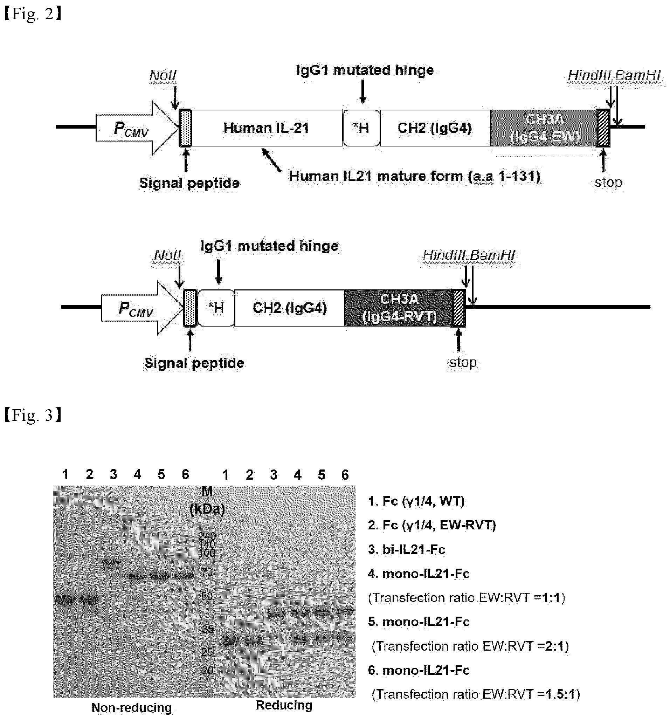

FIG. 2 is a schematic diagram illustrating CH3A (EW) and CH3B (RVT) of expression vectors of Fc (y1/4) for expressing and purifying the fusion protein of FIG. 1A in animal cells.

FIG. 3 shows the results of transiently expressing and purifying the specified wild-type Fc (y1/4, WT), heterodimeric heavy chain constant region Fc (y1/4, EW-RVT), and Bi-IL21-Fc and mono-IL21-Fc (in the mono-IL21-Fc fusion protein, the ratio of CH3A (EW) and CH3B (RVT), expression vectors used for animal cell transformation = 1:1, 1.5:1, 2:1) fusion proteins in HEK293F cells through co-transfection, separating 5 µg of proteins on 10% SDS-PAGE under non-reducing and reducing conditions, and analyzing the size and combination form through Coomasie Blue staining. "Non-reducing" means non-reducing condition, "Reducing" means reducing condition, and "M" means protein marker.

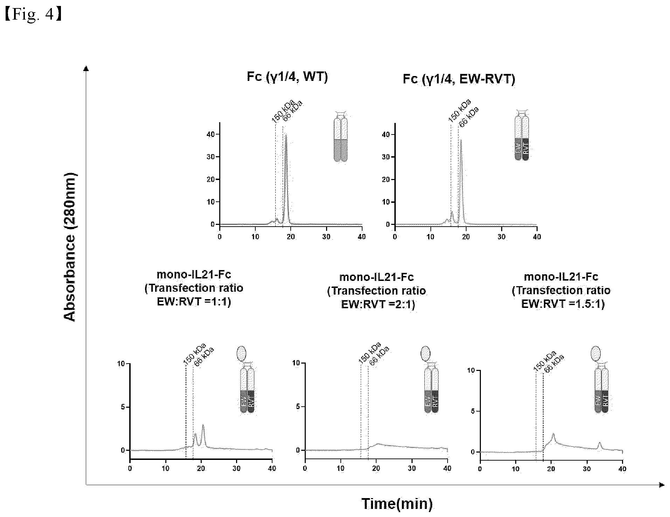

FIG. 4 shows the results of size exclusion chromatography (SEC) analysis of the constructed mono-IL21-Fc fusion protein (ratio of CH3A (EW) and CH3B (RVT), expression vectors used for animal cell transformation = 1:1, 1.5:1, 2:1) along with wild-type Fc (γ1/4, WT) and heterodimeric heavy chain constant region Fc (y1/4, EW-RVT). In the SEC analysis, the running buffer was 1X PBS (pH 7.4, 12 mM phosphate, 137 mM NaCl, 2.7 mM KCl), the flow rate was 0.75 ml/min, and the amount of injected protein was 30 µg (30 µl of injection at 1 mg/ml).

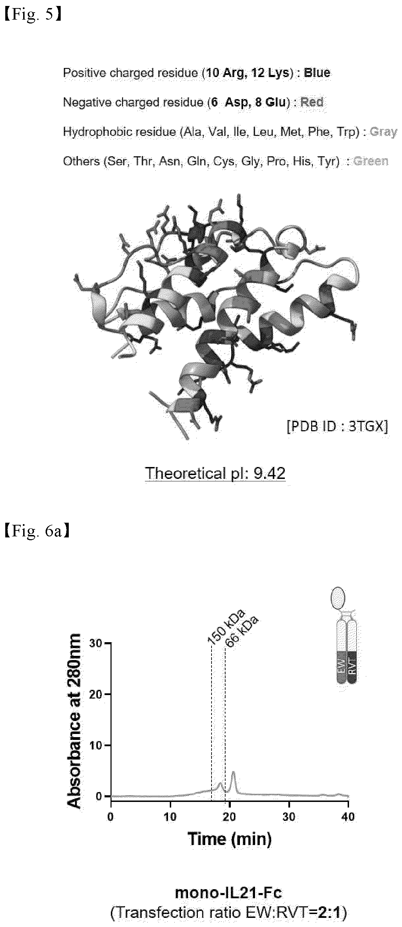

FIG. 5 shows the structure of human IL21 wild type (PDB ID:3tgx) visualized using the Chimera X program wherein each amino acid side chain is highlighted with a designated color depending on characteristics thereof. Blue represents an amino acid with a positively charged side chain, red represents an amino acid with a negatively charged side chain, gray represents an amino acid with a hydrophobic side chain, and green represents other amino acids.

FIG. 6A shows the results of analysis of the constructed mono-IL21-Fc fusion protein using size exclusion chromatography. In the size exclusion chromatography, the running buffer was 1X PBS (pH7.4, 137 mM NaCl), the flow rate was 0.75 ml/min, and the amount of injected protein was 30 µg (30 µl of injection at 1 mg/ml).

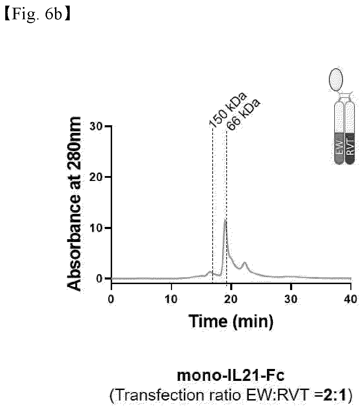

FIG. 6B shows the results of analysis of the constructed mono-IL21-Fc fusion protein using size exclusion chromatography. In the size exclusion chromatography, the running buffer was high salt PBS (pH 7.4, 12 mM phosphate, 2.7 mM KCl, 500 mM NaCl), the flow rate was 0.75 ml/min, and the amount of injected protein was 30 µg (30 µl of injection at 1 mg/ml).

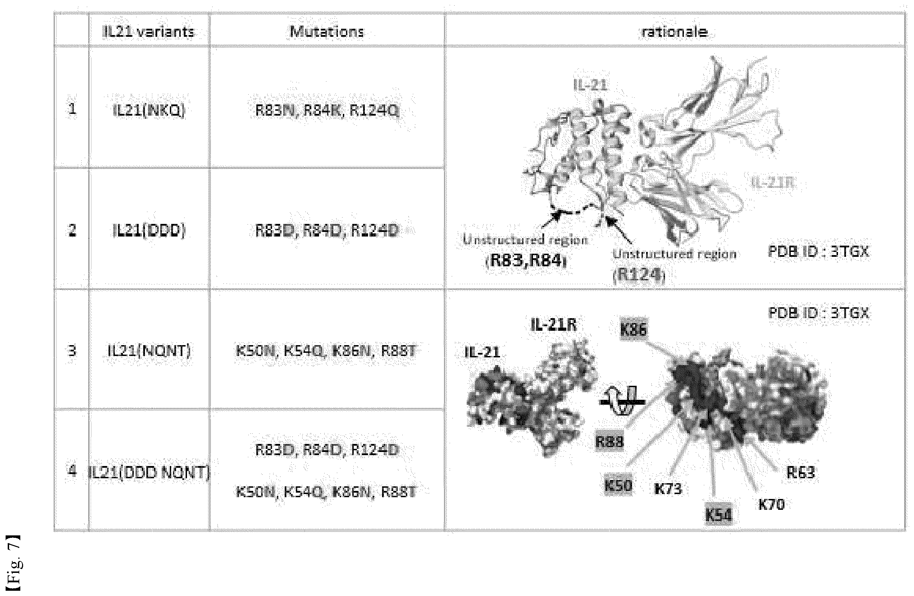

FIG. 7 shows the changed sequences among the sequences of the IL21 amino acid substitution variants constructed in the present invention compared to the human wild type IL21, and images regarding the nomenclature and basis.

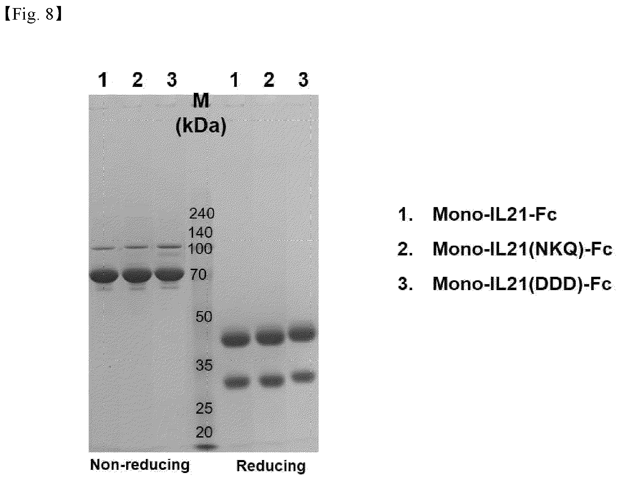

FIG. 8 shows the results of transiently expressing and purifying, as mono-IL21-Fc fusion protein amino acid substitution variants, mono-IL21(NKQ)-Fc and mono-IL21(DDD)-Fc in HEK293F cells through co-transfection, separating 5 µg of proteins along with mono-IL21-Fc fusion protein as a control on 10% SDS-PAGE under non-reducing and reducing conditions, and analyzing the size and combination form through Coomasie Blue staining. "Non-reducing" means non-reducing condition, "Reducing" means reducing condition, and "M" means protein marker.

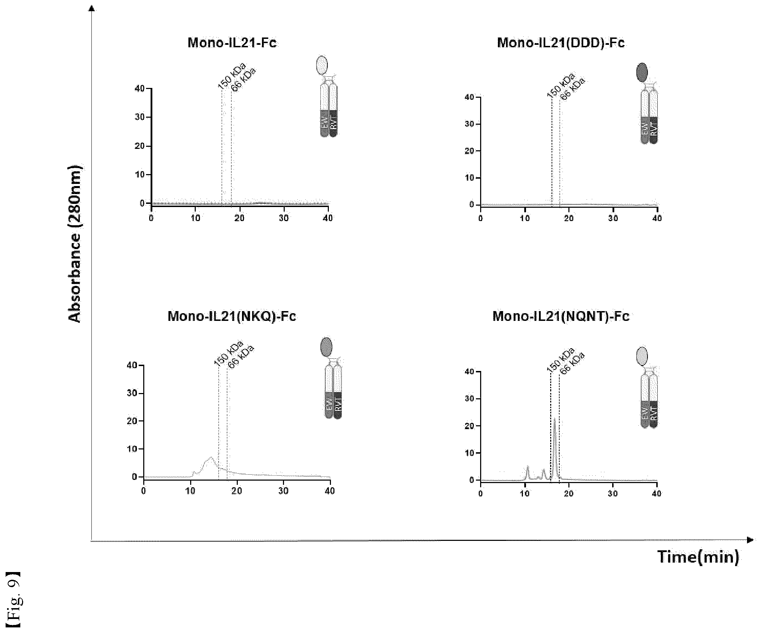

FIG. 9 shows the results of size exclusion chromatography (SEC) analysis of mono-IL21(DDD)-Fc and mono-IL21(NKQ)-Fc, and mono-IL21(NQNT)-Fc, as mono-IL21-Fc fusion protein amino acid substitution variants, along with mono-IL21-Fc fusion protein as a control group. In the SEC analysis, the running buffer was 1X PBS (pH 7.4, 12 mM phosphate, 137 mM NaCl, 2.7 mM KCl), the flow rate was 0.75 ml/min, and the amount of injected protein was 30 µg (30 µl of injection at 1 mg/ml).

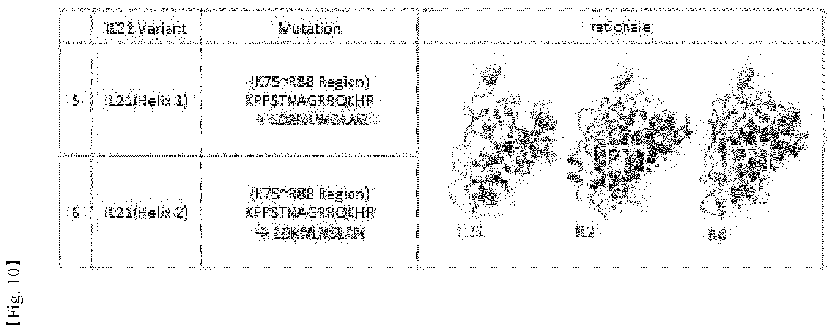

FIG. 10 shows the changed sequences among the sequences of the IL21 ortholog sequence transplant variants constructed in the present invention compared to the human wild type IL21, and images regarding the nomenclature and basis thereof.

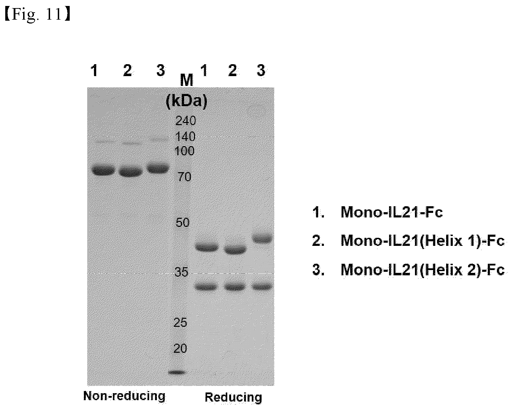

FIG. 11 shows the results of transiently expressing and purifying mono-IL21(Helix 1)-Fc and mono-IL21(Helix 2)-Fc, as mono-IL21-Fc fusion protein ortholog sequence transplant variations, through co-transfection in HEK293F cells, and then separating 5 µg of protein on 10% SDS-PAGE under non-reducing and reducing conditions along with the mono-IL21-Fc fusion protein as a control, and analyzing the size and combination form through Coomasie Blue staining. "Non-reducing" means non-reducing conditions, "Reducing" means reducing conditions, and "M" means a protein marker.

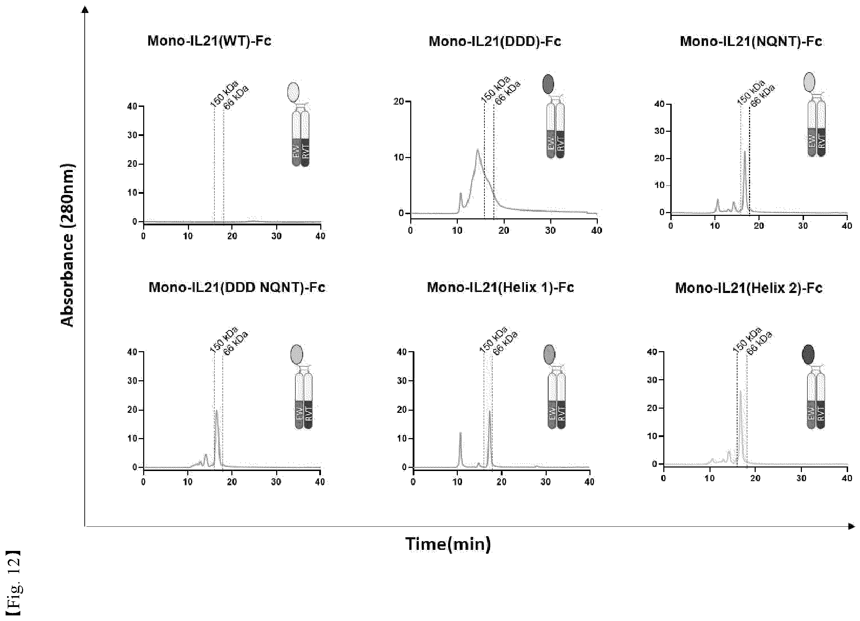

FIG. 12 shows the results of size exclusion chromatography analysis of mono-IL21(Helix 1)-Fc and mono-IL21(Helix 2)-Fc, as mono-IL21-Fc fusion protein ortholog sequence transplant variations, along with mono-IL21-Fc fusion protein, mono-IL21-Fc fusion protein, mono-IL21(DDD)-Fc, mono-IL21 (NQNT)-Fc, and mono-IL21(DDD NQNT)-Fc as controls. In the SEC analysis, the running buffer was 1X PBS, the flow rate of running buffer was 0.75 ml/min, and the amount of injected protein was 30 µg (30 µl of injection at 1 mg/ml).

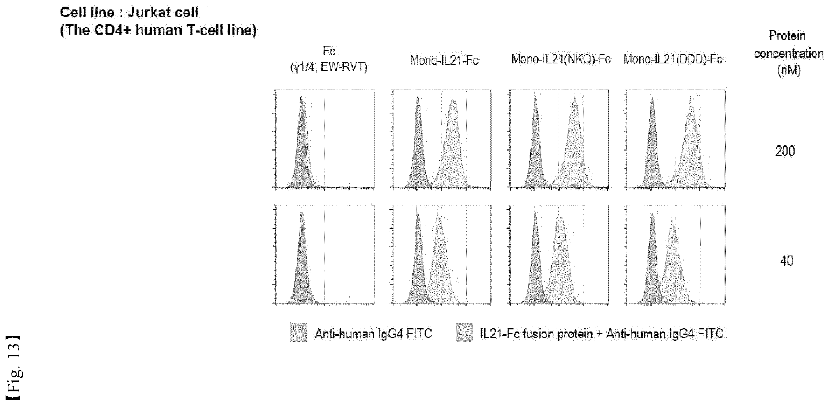

FIG. 13 shows the binding affinity of the IL21 of the mono-IL21-Fc fusion protein and the mono-IL21-Fc fusion protein variant to the IL21 receptor on the cell surface, analyzed using FACS. The Jurkat cell line (human CD4 + T-cell line) expressing the human IL21 receptor was treated with the mono-IL21-Fc fusion protein, mono-IL21 (NKQ)-Fc, mono-IL21 (DDD)-Fc, and wild-type Fc at concentrations of 40 nM and 200 nM, was fluorescently labelled with an anti-human IgG4 antibody and an antibody fused with a fluorescent substance, FITC, and then the fluorescence intensity was measured using FACScalibur.

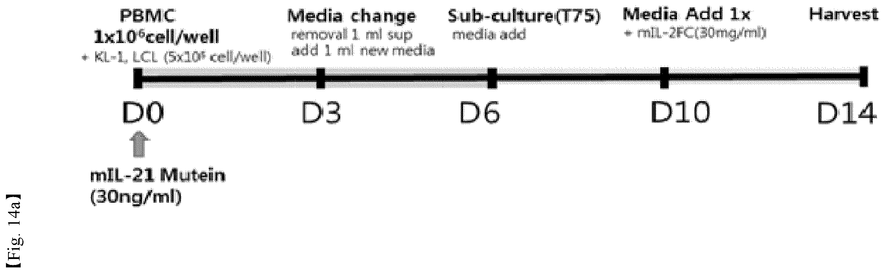

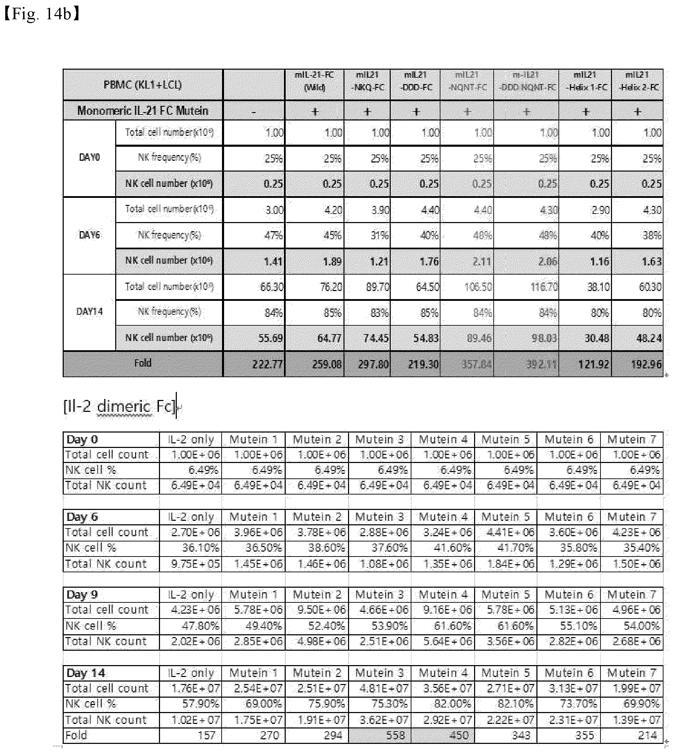

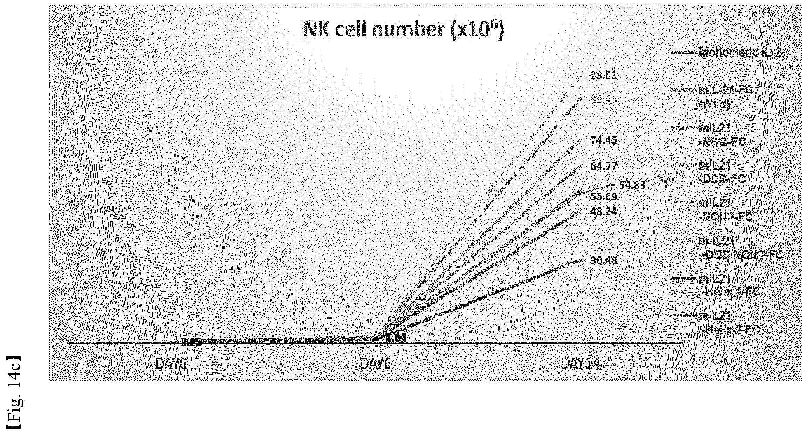

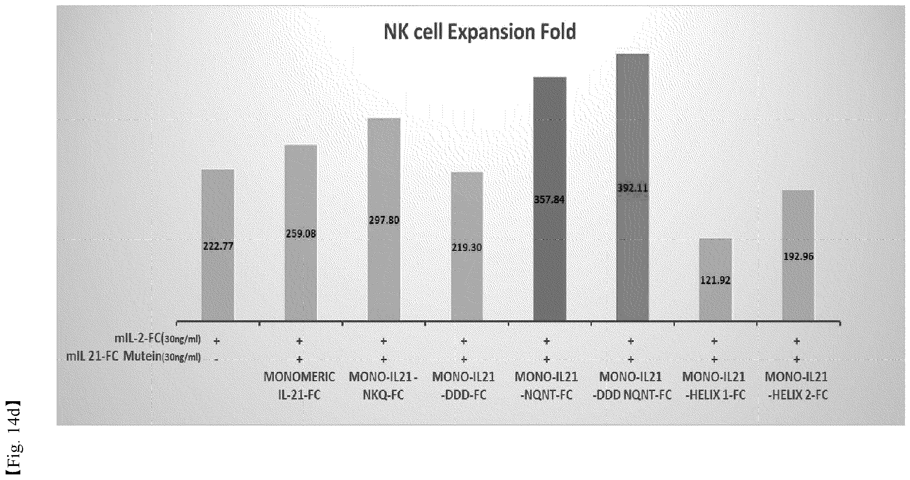

FIG. 14 shows the results identifying effects of the mono-IL21-Fc fusion protein and the mono-IL21-Fc fusion protein variant on proliferation of NK cells. The NK cells were cultured by adding IL-2 (30 ng/ml of monomeric IL-2 Fc) to PBMC isolated from normal blood in the presence of 100 gray gamma-irradiated Jurkat (KL-1) cells and EBV-transformed LCL cells.

FIG. 14A: Timeline of NK cell culture method and schedule.

FIG. 14B: Table showing the yield and percentage of NK cells obtained on Day 0, 6, and 14.

FIG. 14C: Line graph showing the yield of NK cells obtained on Day 0, 6, and 14.

FIG. 14D: Bar graph showing the yield of NK cells obtained on Day 0, 6, and 14.

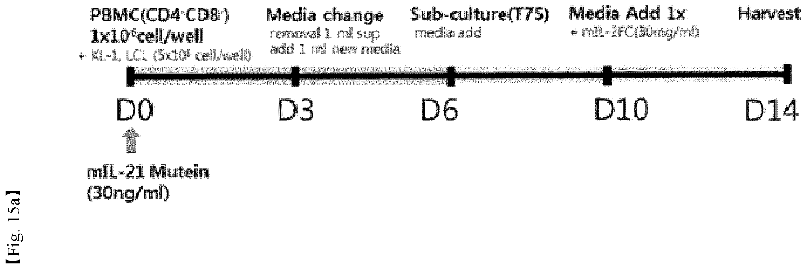

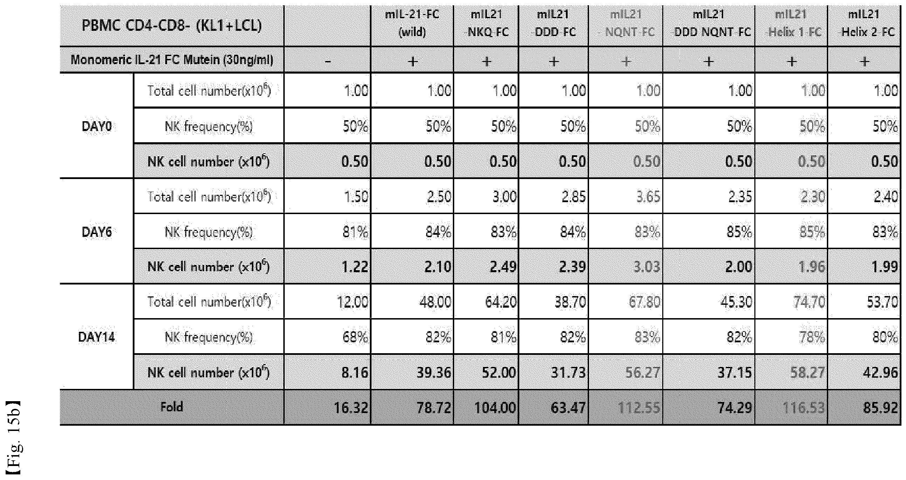

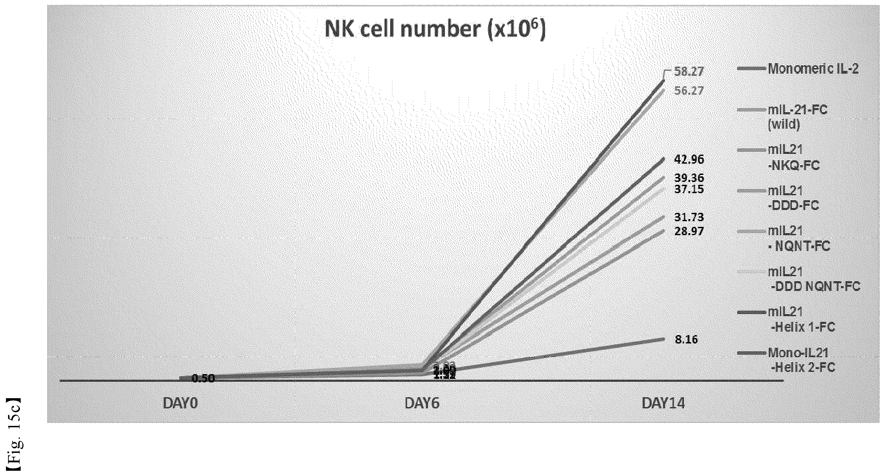

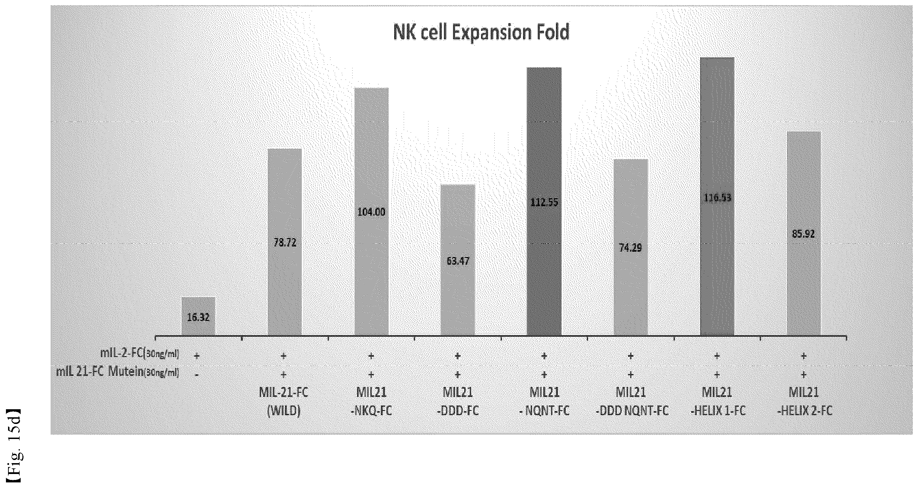

FIG. 15 shows the results identifying effects of the mono-IL21-Fc fusion protein and the mono-IL21-Fc fusion protein variant on proliferation of NK cells. The NK cells were cultured by adding IL-2 (30 ng/ml monomeric IL-2 Fc) to PBMC from which CD4/CD8 T cells are removed in the presence of 100 gray gamma-irradiated Jurkat (KL-1) cells and EBV-transformed LCL cells.

FIG. 15A: Timeline of NK cell culture method and schedule.

FIG. 15B: Table showing the yield and percentage of NK cells obtained on Day 0, 6, and 14.

FIG. 15C: Line graph showing the yield of NK cells obtained on Day 0, 6, and 14.

FIG. 15D: Bar graph showing the yield of NK cells obtained on Day 0, 6, and 14.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In one aspect, the present invention is directed to an interleukin 21 (IL21) variant in which one or more selected from the group consisting of amino acid sequences at positions 50, 54, 75 to 88 and 124 from an N-terminus in an interleukin 21 (IL21) including an amino acid sequence of SEQ ID NO. 4 are substituted or deleted.

The interleukin 21 (IL21) variant may include one or more amino acid substitutions or deletions in human IL21 of SEQ ID NO: 4 including amino acid residues 1 to 131 corresponding to the mature form sequence of human IL21 cytokine (indicated herein as "positions 1-131", excluding the signal peptide and propeptide).

This is accomplished by a method including: (1) selecting an amino acid sequence and region likely to cause deterioration of properties through IL21 structural analysis; (2) designing an amino acid to be substituted to improve properties in the selected amino acid position and region; and (3) fusing the designed IL21 variant with a heterodimeric heavy chain constant region Fc (y1/4, EW-RVT), followed by expressing/purifying and then analyzing the biological properties.

The identification and improvement of the properties of therapeutic protein candidates are important for producing therapeutic proteins. The properties of therapeutic proteins may affect the therapeutic efficacy, pharmacodynamics, and side effects of the proteins. The properties of therapeutic proteins may be reduced due to the net charge, degree of hydrophobicity, and improper folding of the protein in the body. The properties may be deteriorated when the net charge in the body is strongly positive or negative or has a strong hydrophobicity and thus cannot be dissolved in the aqueous environment in the body, or when the protein cannot maintain the proper folding state in the body. This problem may be overcome by selecting and alternately mutating amino acids that cause the deterioration of the properties while maintaining the structure and function of the protein.

The human IL21 has a four-helix (helices A, B, C, to D) structure arranged in an up-up-down-down pattern which is typical of class 1 cytokines and a loop structure connecting the helical structures. In terms of structural characteristics, in the sequence of the mature form of human IL21, amino acids 4 to 23 form helix A, amino acids 41 to 56 form helix B, amino acids 62 to 74 form helix C, and amino acids 102 to 120 form helix D. In binding to the IL21 receptor, 14 IL21 residues and 16 IL21 receptor residues form van der Waals bonds, and thereamong, arginine 3, arginine 7, and glutamine 10 in the IL21 helix A sequence, and arginine 74, and lysine 71 in the helix C sequence are known to be the most important amino acids. In the present invention, in order to improve the *in vivo* biological properties without affecting the structure and function of the wild-type IL21, an amino acid substitution strategy and an ortholog sequence transplant strategy are used.

Specifically, the interleukin-21 variant may include one or more amino acid substitutions or deletions selected from the group consisting of the following amino acid substitutions and deletions in the amino acid sequence of SEQ ID NO: 4:
(1) K50N,
(2) K54Q,
(3) K75L,
(4) P76D,
(5) P77R,
(6) S78N,
(7) T79L,
(8) R83N, R83D, R83L,
(9) R84K, R84D, R84W,
(10) Q85G, Q85S,
(11) K86N, K86L,
(12) H87A,
(13) R88T, R88G, R88N,
(14) R124Q, R124D,
(15) deletion of amino acid at positions 79 to 82, and
(16) deletion of amino acid at positions 81 to 84.

Specifically, the interleukin 21 variant may include one or more amino acid substitutions and/or deletions selected from the group consisting of the following amino acid substitutions and deletions in the amino acid sequence of SEQ ID NO: 4:
(1) R83N, R84K, R124Q;
(2) R83D, R84D, R124D;
(3) K50N, K54Q, K86N, R88T; and
(4) K50N, K54Q, R83D, R84D, K86N, R88T, R124D.

According to the present invention, a mono-IL21-Fc fusion protein ortholog sequence transplant variant was constructed by replacing the sequences of K75 to R88 including the atypical region of IL21 with a complementary human IL4 sequence and an ortholog IL4 sequence. Based on this, the interleukin 21 variant may include one or more amino acid substitutions and/or deletions selected from the group consisting of the following amino acid substitutions and/or deletions in the amino acid sequences of SEQ ID NO: 4:
(1) K75L, P76D, P77R, S78N, and amino acid deletions at positions 79 to 82, R83L, R84W, Q85G, K86L, H87A, R88G; and
(2) K75L, P76D, P77R, S78N, T79L, amino acid deletions at positions 81 to 84, and Q85S, K86L, H87A, R88N.

In a specific embodiment, the interleukin 21 variant may include a sequence selected from the group consisting of sequences of SEQ ID NOS: 5 to 10.

As used herein, the term "mutation (or variation)" refers to a modification of a protein that includes replacing an amino acid with an amino acid having similar biochemical properties that does not cause loss of the structure and biological or biochemical function of the protein. The term "amino acid substitution" refers to a substitution that replaces an amino acid with an amino acid having a side chain that structurally maintains the amino acid but has different chemical properties. Naturally occurring amino acids may be classified into amino acids having side chains that exhibit similar properties, which is well known in the art. These classes include amino acids having basic side chains (e.g., lysine, arginine, and histidine), amino acids having acidic side chains (e.g., aspartic acid and glutamic acid), amino acids having uncharged polar side chains (e.g., glycine, asparagine, glutamine, serine, threonine, tyrosine, and cysteine), amino acids having nonpolar side chains (e.g., alanine, valine, leucine, isoleucine, proline, phenylalanine, methionine, and tryptophan), amino acids having beta-branched side chains (e.g., threonine, valine, and isoleucine) and amino acids having aromatic side chains (e.g., tyrosine, phenylalanine, tryptophan, and histidine). The present invention provides a IL21 variant constructed by amino acid substitution of human IL21, and shows that the IL21 variant retains activity.

As used herein, the term "ortholog sequence transplant" means substituting an amino acid in the same position of IL21 with reference to a specific sequence region of a cytokine (IL-2, IL-4, IL-7, IL-9, IL-15) in the same family as IL21. As a result of structural analysis of IL21, the CD loop structure from Helix C to Helix D and the C-terminal region are atypical regions that are not structurally determined. Although there are various causes for the formation of the atypical region, the biggest cause is the formation of an unstable structure. Therefore, it is presumed that a mutant of IL21, which is constructed by transplanting the sequence of the CD loop, which is expected to form the most stable structure, with reference to the sequence of cytokines in the same family as IL21, into the region, will have improved properties compared to the wild type.

The human IL21 variant of the present invention may include not only the sequence of IL21 variant disclosed herein but also biological equivalents thereto, as long as it can maintain the intended function. For example, additional variations can be made to the amino acid sequence of the antibody in order to further improve the physical properties or other biological properties of the IL21. Such variations include, for example, deletion, insertion and/or substitution of the amino acid sequence residues of the antibody. Such amino acid mutations are based on the relative similarity of amino acid sequence residue substituents, such as the hydrophobicity, hydrophilicity, charge and size thereof. It can be seen through analysis of the size, shape and type of substituents that all of arginine, lysine and histidine are positively charged residues; alanine, glycine and serine have similar sizes; and phenylalanine, tryptophan and tyrosine have similar shapes. Thus, based on these considerations, arginine, lysine and histidine; alanine, glycine and serine; and phenylalanine, tryptophan and tyrosine are considered to be biologically functional equivalents.

In another aspect, the present invention is directed to a fusion protein, as a heterodimer, containing the interleukin 21 variant and an antibody (immunoglobulin) heavy chain constant region (Fc) pair including a first Fc region and a second Fc region, wherein the Fc pair has a mutation in the CH3 domain of the first Fc region or the second Fc region.

The present invention relates to a fusion protein in which interleukin-21 (IL21) cytokine is fused in a monomeric form to a heterodimeric heavy chain constant region Fc of an antibody (hereinafter referred to as "IL21 monomer-Fc fusion protein", "heterodimeric Fc-fused IL21 monomer protein", or "mono-IL21-Fc fusion protein"), or a fusion protein in which a variant of interleukin-21 is fused in a monomeric form to a heterodimeric heavy chain constant region Fc of an antibody (hereinafter referred to as "IL21 monomer-Fc fusion protein variant", "heterodimeric Fc-fused IL21 variant monomer proteins", or "mono-IL21-Fc fusion protein variant").

The present invention also relates to a fusion protein in which interleukin-21 (IL21) cytokine is fused in a dimer form to the antibody heterodimeric heavy chain constant region Fc (heterodimeric Fc) (referred to as "IL21 dimer-Fc fusion protein variant", "heterodimeric Fc-fused IL21 variant dimeric proteins", or "dimer-IL21-Fc fusion protein variant").

As an interleukin-21 variant, a mutant was designed to reduce the non-specific reactivity of IL21 and increase the structural stability through amino acid mutation of wild-type IL21, so that, when the mutant is expressed in the form of a mono-IL21-Fc fusion protein variant, the expression amount of the fusion protein is increased, the formation of undesirable oligomers is reduced under physiological conditions, and the biological properties are improved so that it could exist in the form of a monomer.

Specifically, the present invention provides a technology for developing an IL21 variant that has improved biological properties compared to the mono-IL21-Fc fusion protein, which is a fusion protein of wild-type IL21, but has improved property to preserve the binding ability to IL21 receptors expressed on the cell surface and the proliferation ability of immune cells.

The present invention provides a technology for developing a mono-IL21-Fc fusion protein variant that includes an IL21 variant that can 1) reduce side effects that may occur in the body, 2) improve pharmacokinetics, 3) replace frequent bio-administration required due to the short half-life of the recombinant protein IL21 cytokine, and 4) solve the disadvantage of high cost by reducing nonspecific interactions under physiological conditions of the mono-IL21-Fc fusion protein, which is a fusion protein containing wild-type IL21.

First, a mono-IL21-Fc fusion protein monomeric fusion protein was constructed in which the wild-type IL21 was fused to a heterodimeric heavy chain constant region EW-RVT heterodimeric Fc (γ1/4, EW-RVT) that introduced an EW-RVT mutation based on Fc (y1/4) that has the hinge region sequence of human IgG1 and the CH2-CH3 region of human IgG4. Then, the structure of wild-type IL21 was analyzed, sequences that could affect the physical properties and specificity of the protein were selected, and a mutant in which amino acid units were substituted or the sequences of the same series of cytokines were inserted was designed. This mutant was expressed in the form of a mono-IL21-Fc fusion protein, to develop a mono-IL21-Fc fusion protein variant that can increase the expression amount, reduce the formation of undesirable oligomers under physiological conditions and exist in the form of a monomer.

EW-RVT heterodimer Fc (y1/4, EW-RVT) was used herein, which introduced EW-RVT mutations based on Fc (y1/4) having the hinge region sequence of IgG1 and the CH2-CH3 region of human IgG4. In addition, as a comparative example, Bi-IL21-Fc fusion protein (IL21 dimer-Fc fusion protein) was constructed using wild-type Fc (γ1/4, WT) having the hinge region sequence of human IgG1 and the CH2-CH3 region of human IgG4 and compared.

Based on this, the present invention provides a mono-IL21-Fc fusion protein variant in which the wild-type IL21 amino acid sequence of the mono-IL21-Fc fusion protein is replaced or the sequence of the cytokine of the ortholog is transplanted. More specifically, the mechanism for constructing IL21 for developing the mono-IL21-Fc fusion protein variant is 1) producing a fusion protein with a longer half-life in the body that improves the stability of the substance, reduces non-specific interactions in the body and has a longer half-life in the body by reducing the surface positive charge of the IL21 protein, 2) confirming that the fusion protein mimics the better stability in the body of IL-2 and IL-4 by transplanting a sequence that may cause instability in the IL21 structure into the sequence of IL-2 and IL-4, and comparing the sequences of IL-2 and IL-4, which are cytokines of the same family as IL21, and 3) confirming that the receptor binding ability in a cell line expressing the human IL21 receptor was maintained when compared to the mono-IL21-Fc fusion protein. Based on this mechanism, the present invention has been completed.

The heterodimeric Fc-fused protein variants containing interleukin 21 variants according to the present invention provide proteins that can maintain functions and activity of the conventional mono-IL21-Fc fusion proteins by maintaining the structure thereof.

As used herein, the "Fc region" refers to a region including a CH2 domain, a CH3 domain and a hinge domain derived from an antibody. As used herein, the expression "the first Fc region and the second Fc region are mutated to promote the formation of a heterodimeric heavy chain constant region (heterodimeric Fc)" means that antibodies present in the natural system have a homodimeric heavy chain constant region (homodimeric Fc) form in which two Fc regions have the same sequence and, by inducing a mutation in a part of the sequence of this Fc region, the formation of a heterodimeric heavy chain constant region (heterodimeric Fc) is promoted through a specific non-covalent bond between the first Fc region and the second Fc region, and the formation of the homodimer is reduced or, preferably, hardly occurs.

Preferably, the mutation that promotes the formation of a heterodimeric heavy chain constant region (heterodimeric Fc) of the first Fc region and the second Fc region according to the present invention may include a mutation that promotes the formation of a heterodimeric heavy chain constant region (heterodimeric Fc) of each of the CH3 domains included in the first Fc region and the second Fc region derived from the antibody.

In the present invention, the heterodimeric heavy chain constant region includes a first Fc region and a second Fc region, and the first Fc region and the second Fc region are characterized in that the CH3 domain is mutated to promote the formation of the heterodimeric heavy chain constant region.

The CH3 domain mutation of the first Fc region or the second Fc region includes at least one mutation selected from the following mutations and the mutation position is determined in accordance with the EU index:
(1) substitution at position K370 in the CH3 domain of the first Fc region with K370E, K370R, K370M, K370D or K370H;
(2) substitution at position K409 in the CH3 domain of the first Fc region with K409W;
(3) substitution at position E357 with E357N, E357D, E357A, E357I, E357G or E357M, and substitution at position S364 with S364T or S364W in the CH3 domain of the second Fc region; and
(4) substitution at position F405 with F405T, and substitution at position D399 with D399V in the CH3 domain of the second Fc region.

The CH3 domain of the first Fc region or the second Fc region may further include the following substituted amino acid and the mutation position is determined in accordance with the EU index:
(i) cysteine (C) substituted at position Y349 in the CH3 domain of the first Fc region; or
(ii) cysteine (C) substituted at position S354 in the CH3 domain of the second Fc region.

Specifically, the mutation of the CH3 domain of the first Fc region or the second Fc region may include one or more mutations selected from the following mutations (provided that the mutation position is determined in accordance with the EU index):
(1) substitution of an amino acid residue at position K360 in the CH3 domain of the first Fc region with K360E;
(2) substitution of an amino acid residue at position E347 in the CH3 domain of the second Fc region with E347R;
(3) Substitution of amino acid residue at position K409 in the CH3 domain of first Fc region with K409W; and
(4) Substitution of amino acid residue at position F405 with F405T and substitution of amino acid residue at position D399 with D399V in the CH3 domain of the second Fc region.

The CH3 domain of the first Fc region or the second Fc region may further include the following substituted amino acid, and the mutation position may be determined in accordance with the EU index:
(i) cysteine (C) substituted at position Y349 in the CH3 domain of the first Fc region; or
(ii) cysteine (C) substituted at position S354 in the CH3 domain of the second Fc region.

In a specific embodiment according to the present invention, the CH3 domain mutation of the first Fc region and the second Fc region include: (1) substitution at position K360 in the CH3 domain of the first Fc region with K360E, substitution at position K409 in the CH3 domain of the first Fc region with K409W; and (2) substitution at position E347 with E347R, substitution at position F405 with F405T and substitution at position D399 with D399V in the CH3 domain of the second Fc region, and the mutation position is determined in accordance with the EU index (hereinafter referred to as "EW-RVT").

As used herein, the term "EW-RVT heterodimeric Fc" means EW-RVT heterodimeric Fc (γ1/4, EW-RVT) with the EW-RVT mutation based on Fc (y1/4) having the hinge region sequence of IgG1 and the CH2-CH3 region of human IgG4 introduced thereto.

Preferably, the CH3 domain included in the antibody-derived first Fc region and the second Fc region according to the present invention may have a sequence selected from the group consisting of amino acid sequences represented by SEQ ID NO: 1 and SEQ ID NO: 2.

The first Fc region and the second Fc region may be derived from an Fc region selected from the group consisting of human IgG1, IgG2, IgG3, IgG4, IgM, IgA, IgD and IgE, preferably, the first Fc region and the second Fc region may be derived from IgG1, IgG2, IgG3, or IgG4, and the first Fc region and the second Fc region may be derived from an isotype antibody.

In particular, the first Fc region and the second Fc region derived from the antibody according to the present invention preferably have the sequence of the CH3 domain derived from IgG4 described in Table 1.

As used herein, the term "γ1/4" means that an antibody heavy chain constant region domain in which the hinge region uses an IgG1 isotype that is widely adopted in commercial therapeutic antibodies due to its high stability, and the CH2 and CH3 regions use an IgG4 isotype that prevents the occurrence of antibody-specific functions such as unwanted ADCC (antibody-dependent cellular cytotoxicity)/CDC (complement-dependent cytotoxicity), and an Fc fused thereto is referred to as "Fc(γ1/4)".

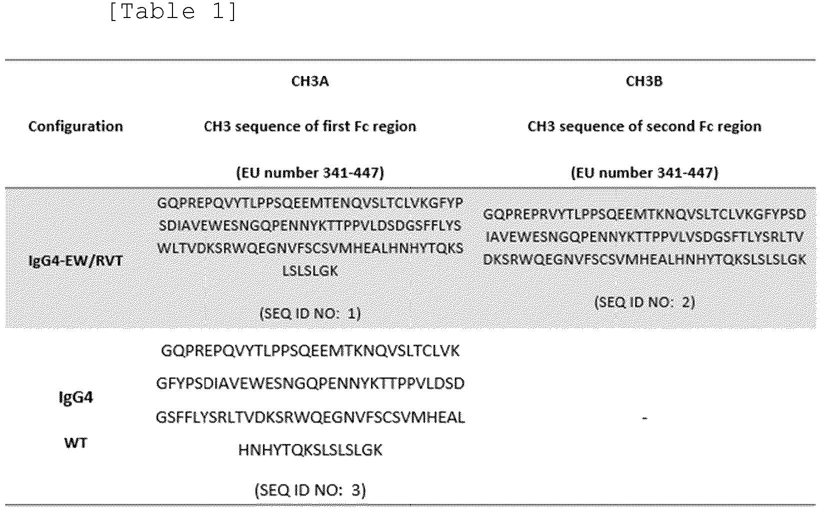

Specifically, the fusion protein according to the present invention may include an interleukin 21 variant including a sequence selected from the group consisting of sequences of SEQ ID NOS: 5 to 10, and an antibody (immunoglobulin) heavy chain constant region (Fc) pair including a first Fc region including a sequence of SEQ ID NO: 1, and a second Fc region including a sequence of SEQ ID NO: 2.

In another aspect, the present invention is directed to a nucleic acid encoding the interleukin 21 variant or fusion protein. The nucleic acid encoding the interleukin 21 variant or fusion protein of the present invention may be isolated to recombinantly produce the interleukin 21 variant or fusion protein.

As used herein, the term "nucleic acid" is intended to encompass both DNA (gDNA and cDNA) and RNA molecules, and nucleotides, which are basic constituent units of the nucleic acid, include naturally derived nucleotides as well as analogues having modified sugar or base moieties. The sequence of nucleic acid encoding the IL21 variant according to the present invention may be varied. Such variation includes addition, deletion, or non-conservative substitution or conservative substitution of nucleotides.

The DNA encoding the interleukin 21 variant or fusion protein may be easily isolated or synthesized using conventional molecular biological techniques (for example, by using an oligonucleotide probe that can specifically bind to CH3A (EW) and CH3B (RVT) DNA), and the nucleic acid is isolated, inserted into a replicable vector, further cloned (amplified with DNA) or further expressed. Based on this, in another aspect, the present invention is directed to a recombinant expression vector containing the nucleic acid.

As used herein, the term "expression vector" refers to a means for expressing target genes in host cells, and includes plasmid vectors, cosmid vectors, and viral vectors such as bacteriophage vectors, adenovirus vectors, retroviral vectors and adeno-associated viral vectors. Vector components generally include, but are not limited to, one or more of the following components: signal sequences, replication origins, one or more antibiotic resistance marker genes, enhancer elements, promoters, and transcription termination sequences. The interleukin 21 variant or nucleic acid encoding the antibody is operably linked to promoters, transcription termination sequences or the like.

The term "operably linked" means functional linkage between a nucleic acid expression regulation sequence (e.g., an array of the binding site of the promoter, signal sequence or transcription regulator) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of mammalian cells (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

In another aspect, the present invention is directed to a host cell transformed with the recombinant expression vector. The host cell used to produce the antibody of the present invention may be a prokaryote, yeast or higher eukaryotic cell, but is not limited thereto.

Prokaryotic host cells such as *Escherichia coli,* strains of the genus *Bacillus,* such as *Bacillus subtilis* and *Bacillus thuringiensis, Streptomyces spp., Pseudomonas spp.* (for example, *Pseudomonas putida*)*, Proteus mirabilis* and *Staphylococcus spp.* (for example, *Staphylococcus carnosus*) may be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

As used herein, the term "fusion" means integrating two molecules having different or the same functions or structures and may be carried out by any physical, chemical or biological method that can bind an antibody (immunoglobulin) heavy chain constant region (Fc) pair including a first Fc region and a second Fc region to the interleukin 21 variant. Fusion is preferably performed by a linker peptide and the linker peptide can mediate the fusion with the bioactive molecule at various positions of the interleukin 21 variant of the present invention and the antibody (immunoglobulin) heavy chain constant region (Fc) pair including a first Fc region and a second Fc region.

The interleukin-21 variant may bind to the N-terminus of the first Fc region or the second Fc region. The interleukin-21 variant may be fused to the N-terminus of either the first Fc region or the second Fc region in a monomeric form. A mutant that reduces the non-specific reactivity of IL21 and increases structural stability is designed through an amino acid mutation of wild-type IL21, and when the mutant is expressed in the form of a mono-IL21-Fc fusion protein variant, the expression amount of the fusion protein is increased, the formation of undesirable oligomers is reduced under physiological conditions, and the biological properties are improved so that it can exist in the form of a monomer.

The IL-21 or variant thereof may be linked to the first Fc region or the second Fc region by a linker. The linker may be a peptide linker and may have a length of about 10-25 aa. For example, the linker may include hydrophilic amino acids such as glycine and/or serine, but is not limited thereto.

Specifically, the linker may include, for example, (GS)n, (GGS)n, (GSGGS)n or (GnS)m (wherein n and m are each 1 to 10), but the linker may be, for example, (GnS)m (wherein n and m are each 1 to 10).

In another aspect, the present invention is directed to a composition for preventing or treating cancer containing the interleukin 21 variant or fusion protein as an active ingredient.

In another aspect, the present invention is directed to a method of preventing or treating cancer including administering the interleukin 21 variant or fusion protein to a patient.

In particular, the mono-IL21-Fc fusion protein variant according to the present invention activates the proliferation of T cells and/or NK cells and increases toxicity, thereby exhibiting an antitumor or anticancer effect. The mono-IL21-Fc fusion protein variant according to the present invention is used for the treatment of diseases related to cancer.

For example, the cancer may be selected from the group consisting of colon cancer, melanoma, breast cancer, pancreatic cancer, kidney cancer, prostate cancer, ovarian cancer, small intestine cancer, esophageal cancer, cervical cancer, lung cancer, lymphoma, and blood cancer.

In another aspect, the present invention is directed to a composition for culturing immune cells containing the interleukin 21 variant or fusion protein according to the present invention. In another aspect, the present invention is directed to a culturing method including treating immune cells with the interleukin 21 variant or fusion protein according to the present invention.

In some cases, the composition may further contain one selected from the group consisting of a medium, a serum, a supplement, and a combination thereof. The medium may include one selected from the group consisting of amino acids, sugars, inorganic salts, and vitamins. Preferably, the cell culture medium may include all of amino acids, sugars, inorganic salts, and vitamins.

The composition contains a medium used for culturing cells, and specifically includes a medium for culturing NK cells, more specifically CD3-CD56+ cells. The composition contains ingredients required for cell growth and survival *in vitro,* or ingredients that facilitate cell growth and survival. Specifically, the ingredients may include vitamins, essential or non-essential amino acids, and trace elements.

The medium contains amino acid components, vitamin components, inorganic salt components, other components, and purified water,
a) The amino acid ingredient includes at least one amino acid or a combination thereof selected from the group consisting of glycine, L-alanine, L-valine, L-leucine, L-isoleucine, L-threonine, L-serine, L-cysteine, L-methionine, L-aspartic acid, L-asparagine, L-glutamic acid, L-glutamine, L-lysine, L-arginine, L-histidine, L-phenylalanine, L-tyrosine, L-tryptophan, L-proline, β-alanine, gamma-aminobutyric acid, ornithine, citrulline, homoserine, triiodotyrosine, thyroxine and deoxyphenylalanine, preferably, includes at least one amino acid or a combination thereof selected from the group consisting of glycine, L-alanine, L-arginine, L-cysteine, L-glutamine, L-histidine, L-lysine, L-methionine, L-proline, L-serine, L-threonine and L-valine, and
b) the vitamin component may include at least one vitamin or a combination thereof selected from the group consisting of biotin, calcium D-pantothenate, folic acid, niacinamide, pyridoxine hydrochloride, riboflavin, thiamine hydrochloride, vitamin B12, choline chloride, i-inositol and ascorbic acid, and preferably includes at least one vitamin or a combination thereof selected from the group consisting of i-inositol, thiamine hydrochloride, niacinamide, and pyridoxine hydrochloride,
c) the inorganic salt ingredient includes at least one inorganic salt or a combination thereof selected from the group consisting of calcium chloride (CaCl₂) (anhydrous), copper sulfate pentahydrate (CuSO₄-5H₂O), ferric sulfate heptahydrate (FeSO₄·7H₂O), magnesium chloride (anhydrous), magnesium sulfate (MgSO₄) (anhydrous), potassium chloride (KCl), sodium chloride (NaCl), disodium hydrogen phosphate (Na₂HPO₄), sodium hydrogen phosphate monohydrate (NaH₂PO₄·H₂O), zinc sulfate heptahydrate (ZnSO₄·7H₂O), ferric nitrate nonahydrate (Fe(NO₃)₃·9H₂O), and sodium bicarbonate (NaHCO₃), preferably, includes at least one inorganic salt or a combination thereof selected from the group consisting of sodium chloride (NaCl), sodium bicarbonate (NaHCO₃), potassium chloride (KCl), calcium chloride (CaCl₂) (anhydrous), and sodium bicarbonate monohydrate (NaH₂PO₄·H₂O), and
d) the other ingredient includes at least one or a combination thereof selected from the group consisting of D-glucose (dextrose), sodium pyruvate, hypoxanthine Na, thymidine, linoleic acid, lipoic acid, adenosine, cytidine, guanosine, uridine, 2'-deoxyadenosine, 2'-deoxycytidine HCl and 2'-deoxyguanosine, preferably, sodium pyruvate.
e) Purified water is used to dissolve the amino acids, vitamins, inorganic salts and other ingredients, and may be obtained through one or more distillations or purified through a filter.

In addition, the composition may further contain a growth factor or a cytokine. The growth factor may be IGF, bFGF, TGF, HGF, EGF, VEGF, or PDGF alone or in combination thereof, but is not particularly limited thereto. The cytokine may be IL-1, IL-4, IL-6, IFN-γ, IL-10, IL-15, IL-17, or IL-21 alone or in combination thereof, but is not particularly limited thereto.

In addition, the composition may further contain an antibody for activating natural killer cells. The antibody for activating natural killer cells may be an anti-CD3 antibody, an anti-CD2 antibody, an anti-CD335 antibody or the like alone or in combination thereof, but is not particularly limited thereto. In addition, the composition may contain a bead to which the antibody for activating natural killer cells is bound.

In particular, the medium composition for culturing NK may further contain one selected from the group consisting of IL-15, IL-21, and combinations thereof.

The IL-15 and IL-21 are a type of interleukin (IL), and refer to proteinaceous biologically active substances produced by immune cells such as lymphocytes, monocytes, and macrophages. IL-15 and IL-21 promote the proliferation of natural killer cells and may be used when culturing natural killer cells using monocytes as raw material cells. However, when IL-15 and IL-21 are used alone or in combination thereof, there are problems of low proliferation rate and purity (Biossel L. et al., Biology of Blood and Marrow Transplantation, 14, 1031-1038, 2008).

Specifically, the medium may be an animal cell culture medium such as DMEM (Dulbecco's Modified Eagle Medium), EDM (endothelial differentiation medium), MEM (minimal essential medium), BME (basal medium eagle), RPMI 1640, F-10, F-12, a-MEM (a-minimal essential medium), G-MEM (Glasgow's minimal essential medium), Iscove's modified Dulbecco's medium, AIM-V medium, X-vivo^{™} 15 medium, or NK MACS medium. In one embodiment of the present invention, the medium may be AIM-V medium, X-vivo^{™} 15 medium or NK MACS medium.

As used herein, the term "serum" refers to a transparent supernatant separated from blood after the blood is completely coagulated. In addition, serum should be added to the synthetic medium for culturing animal cells, and is generally serum from cows, horses, or humans. The bovine serum may be selected from fetal bovine serum (FBS), newborn bovine serum, calf serum, or maternal bovine serum depending on the time of blood collection. The human serum may be human serum from a donor with blood type AB and may be human AB serum that can minimize immune reactivity due to absence of antibodies to blood type A and B antigens. In addition, CTS immune cell SR, or the like may be used as a substitute for the "serum". In one embodiment of the present invention, human AB serum or CTS Immune Cell SR was used.

The supplement may be GlutaMAX (Gibco^{™}), an L-glutamine substitute, to improve stability and cell activity during cell culture. In addition, the supplement may be NK MACS supplement (Miltenyi Biotec, 130-113-102).

The immune cell may include at least one selected from the group consisting of T cells, NK cells, cytokine-induced killer cells (CIKs), activated cytotoxic T lymphocytes (CTLs), macrophages, tumor-infiltrating lymphocytes (TILs), and dendritic cells.

Immune cells, particularly NK cells, produced by the method described above are provided. A pharmaceutical composition for preventing or treating cancer containing the NK cells is provided. The dosage of the pharmaceutical composition may be adjusted depending on various factors including the type of disease, the severity of the disease, the types and contents of the active ingredient and other ingredients included in the composition, the type of formulation, and the patient's age, weight, general health, gender and diet, administration time, administration route and secretion rate of the composition, treatment period, and concurrently used drugs.

The dosage of the pharmaceutical composition may be 1×10² cells/kg to 1.0 × 10¹³ cells/kg, or 1×10⁷ cells/kg to 1.5 × 10¹¹ cells/kg, based on the active ingredient, NK cells. At this time, administration may be performed once a day, or several times in divided portions.

Hereinafter, the present invention will be described in more detail with reference to examples. However, it will be obvious to those skilled in the art that these examples are provided only for illustration of the present invention, and should not be construed as limiting the scope of the present invention.

### Example 1. Construction of mono-IL21-Fc fusion protein

The Fc variant for construction of the fusion protein used herein was a heterodimeric heavy chain constant region Fc (γ1/4, EW-RVT) based on IgG4, in which a EW-RVT mutation was introduced to form a heterodimer. According to previous reports, in the construction of immunocytokines, which are fusion forms of antibodies and cytokines, the antibody-specific functions of IgG1, such as ADCC/CDC, rather promote clearance *in vivo.* An IgG4 isoform that has almost no ADCC/CDC function compared to IgG1, was used for the construction of all fusion proteins specified herein (Gillies SD et al., 1999). In particular, human IL21 was linked to the heavy chain constant region using the hinge region without an additional peptide linker to facilitate interaction with the IL21 receptor. The hinge region used here was a widely used IgG1-derived hinge and the cysteine residues in the upper hinge region excluding the cysteine residues in the core hinge region for duplex formation were substituted with serine residues to prevent undesired disulfide bonds from forming during protein fusion (Mutated hinge, FIG. 2).

Among the isotype-specific variants expected to maintain heterodimer formation ability similar to that of the previously reported IgG1-based EW-RVT variants, a human IL21 (Interleukin 21, Table 2, SEQ ID NO: 5) fusion protein was constructed using an IgG4-based variant (IgG4-EW-RVT, Choi et al, 2013; Korean Patent No. 10-1522954). IL21, which exists in the natural world, is a cytokine that acts as a monomer, and is active when one IL21 binds to one IL21 receptor (IL21R) and one γc-chain. Therefore, one IL21 was linked to only one of the different Fc variants (CH3A or CH3B) using the heterodimeric heavy chain constant region Fc (y1/4, EW-RVT) to maintain the monomeric form of IL21 existing in the natural world.

FIG. 1A is a schematic diagram illustrating the mono-IL21-Fc fusion protein into which the CH3 mutant pair was introduced. The DNA encoding IL21-Fc (γ1/4,EW) into which the CH3 mutant pair was introduced was cloned in-frame into pcDNA3.1(+) (Invitrogen, USA), an animal cell expression vector having a CMV promoter, using NotI/HindIII, to form a signal sequence-IL21 mature form-mutated hinge-CH2-CH3A. The IL21 used here was human IL21 (Uniprot entry name, Q9HBE4; sequence number 4), and only the DNA sequence encoding the mature form excluding the signal sequence was used.

As a comparative example for the mono-IL21-Fc fusion protein, Bi-IL21-Fc fused with wild-type Fc was constructed. In a similar manner as described above, the DNA sequence encoding human IL21 mature form was cloned in-frame into pcDNA3.1(+) (Invitrogen, USA), an animal cell expression vector containing wild-type IgG4 CH3, using NotI/HindIII restriction enzymes, to form a structure of signal sequence-IL21 mature form-Mutated Hinge-CH2-CH3. Table 2 shows the amino acid sequence of human IL21 used for constructing the mono-IL21-Fc fusion protein and Bi-IL21-Fc fusion protein. The amino acid sequence was constructed as a 131 amino acid peptide of the mature form of IL21 (amino acid No. 32 to amino acid No. 162 of human IL21) (Parrish-Novak et al. Nature, 2000;408(6808):57-63), and the numbering thereof is the same as that of the previous study on mono-IL21-Fc (Korean Patent No. 10-1928981; U.S. Patent Registration No. US10800825B2). That is, herein, residues 32 to 1 of the mature form of IL21 are referred to as residues 1-131 (see Table 2 and Table 4).

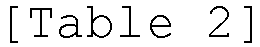

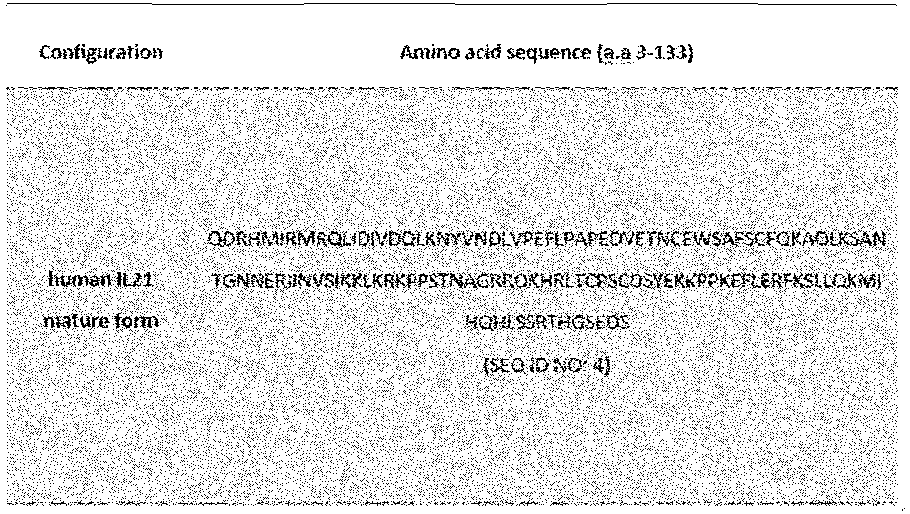

### Example 2. Construction of Fc and mono-IL21-Fc fusion proteins and expression/purification

Expression of wild-type Fc (γ1/4, WT) and heterodimeric heavy chain constant region Fc (y1/4, EW-RVT), Bi-IL21-Fc fusion protein, and mono-IL21-Fc fusion protein (mono-IL21-Fc fusion protein has a ratio of 1:1, 1.5:1, 2:1 of expression vectors of CH3A (EW) and CH3B (RVT) fused with human IL21) was performed by transiently transfecting a mixture of a plasmid encoding each protein and polyethylenimine (PEI) (Polyscinece) into HEK293-F (Invitrogen) cells and incubating the result in a shaking flask containing serum-free FreeStyle^{™} 293 expression medium (Invitrogen). The details of the method are as follows:

For transient transfection, HEK293-F cells were seeded at a density of 1.0 × 10⁶ cells/ml in 90 ml of medium in a shaker flask (Corning) and incubated at 135 rpm in 8% CO₂. After 24 hours, to produce fusion proteins including each heterodimeric heavy chain constant region, the corresponding CH3A and CH3B plasmids were diluted in 4 ml FreeStyle^{®} 293 expression medium (Invitrogen) at a total of 125 µg (1.25 µg/ml), more specifically, at 125 µg for Fc (γ1/4, WT), at 62.5 µg CH3A and 62.5 µg CH3B for the heterodimeric heavy chain constant region Fc (γ1/4, EW-RVT), at 125 µg for bi-IL21-Fc, at 62.5 µg of CH3A and 62.5 µg of CH3B for mono-IL21-Fc fusion protein CH3A (EW):CH3B(RVT)=1:1, at 75 µg of CH3A and 50 µg of CH3B for CH3A (EW):CH3B(RVT)=1.5:1, 83.3 µg of CH3A and 41.7 µg of CH3B for CH3A (EW): CH3B (RVT) = 2:1, was filtered, mixed with 6 ml of medium (3.75 µg/ml) in which 375 µg of PEI was diluted, and reacted at room temperature for 10 minutes. Then, the reacted mixed medium was added to the cells seeded in 90 ml in advance and cultured for 5 days at the shortest and 7 days at the longest. As a result, the proteins produced by the cells are secreted outside the cells and accumulated in the medium. Therefore, the proteins were purified using a protein A Sepharose column (GE healthcare) from the cell culture supernatant collected by centrifugation at 3800 rpm for 25 minutes after cell culture. At this time, the purification was performed in accordance with the standard protocol provided by the protein A column company, and the eluted protein was concentrated after exchanging the buffer with 1X PBS using a Vivaspin 30,000 MWCO (Satorius) centrifugal concentrator. Absorbance of the purified protein was measured at a wavelength of 562 nm using a solution in the BCA protein assay kit (Thermo) and assayed in accordance with the drawn standard curve. Table 3 shows the purification yields of the expressed wild-type Fc(γ1/4, WT), heterodimeric heavy chain constant region Fc(y1/4, EW-RVT), mono-IL21-Fc fusion protein (ratio of CH3A (EW): CH3B (RVT) expression vector used during transfection = 1:1, 1.5:1, 2:1), and Bi-IL21-Fc. Experiments were independently performed in triplicate and the results were expressed as the standard error of the mean (mean ± SD). The result of the yield verification showed that the mono-IL21-Fc fusion protein had a significantly higher yield compared to the Bi-IL21-Fc fusion protein.

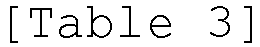

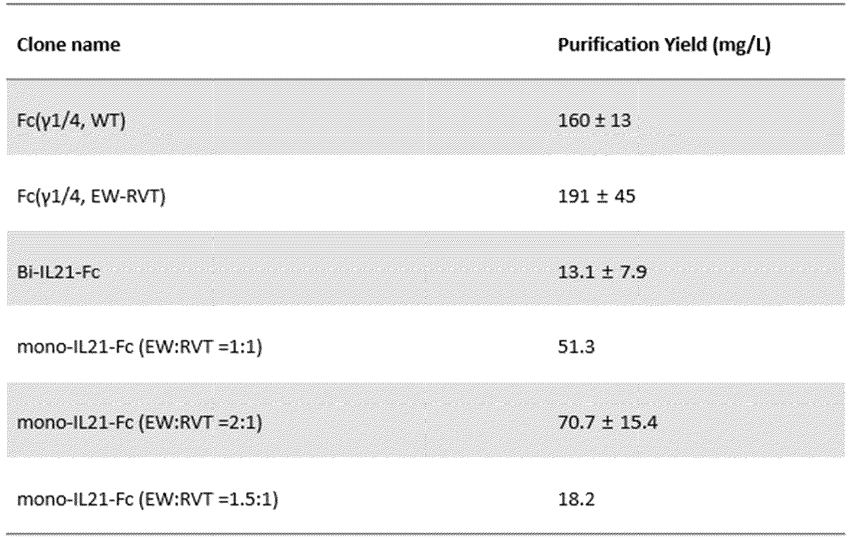

The purified wild-type Fc (γ1/4, WT) and heterodimeric heavy chain constant region Fc (γ1/4, EW-RVT), mono-IL21-Fc fusion protein (expression vector ratio of CH3A (EW) and CH3B (RVT) = 1:1, 1.5:1, 2:1 at the time of transfection) and Bi-IL21-Fc fusion protein (5 µg) were analyzed through SDS-PAGE under 10% non-reducing and reducing conditions (FIG. 3). Under non-reducing conditions, mono-IL21-Fc fusion protein was observed at 71 kDa, and Bi-IL21-Fc fusion protein was observed at 86 kDa. Under reducing conditions, Bi-IL21-Fc had one band at 43 kDa, and mono-IL21-Fc fusion protein which includes two plasmids including IL21-fused heterodimeric heavy chain constant region CH3A (EW) and heterodimeric heavy chain constant region CH3B (RVT) had two bands at 43 and 28 kDa. The heterodimeric heavy chain constant regions CH3A and CH3B were each expected to have a protein size of about 25 kDa, and human IL21 was expected to have a protein size of 15 kDa, but bands larger than the expected protein size were observed under reducing and non-reducing conditions due to N-glycosylation in human IL21 and Fc. The SDS-PAGE results of the mono-IL21-Fc fusion protein under non-reducing conditions varied depending on the plasmid ratio. At EW:RVT=1:1 and 1.5:1, the mono-IL21-Fc fusion protein, in which the heterodimeric heavy chain constant regions, CH3A (EW) and CH3B (RVT) are mixed in a ratio of 1:1, was not formed, a band that appeared to be a dimer formed by two monomers of the heterodimeric heavy chain constant region CH3B (RVT) was observed, and such a band was not observed at EW:RVT=2:1. Therefore, the mono-IL21-Fc fusion protein and mono-IL21-Fc fusion protein variants described below were all constructed at a plasmid ratio of EW:RVT=2:1.

### Example 3. Evaluation of properties of mono-IL21-Fc fusion protein

FIG. 4 is a chromatogram showing the mAU at 280 nm as a function of time (minutes) upon SEC (size exclusion chromatography) to analyze physical properties of the expressed wild-type Fc (γ1/4, WT), heterodimeric heavy chain constant region Fc (γ1/4, EW-RVT), and mono-IL21-Fc fusion protein (expression vector ratio of heterodimeric heavy chain constant region CH3A (EW): CH3B (RVT) = 1:1, 1.5:1, 2:1 upon transformation). The measurements were performed using an HPLC (Agilent Technologies, USA) instrument and a Superdex200 10/300 GL column (Cytiva, USA) according to the following specific protocol.

Tertiary water that has undergone filtration and degassing, and 1X PBS (pH 7.4, 12 mM phosphate, 137 mM NaCl, 2.7 mM KCl) were prepared. Wild-type Fc (γ1/4, WT), heterodimeric heavy chain constant region Fc (γ1/4, EW-RVT), Bi-IL21-Fc, and mono-IL21-Fc fusion proteins (plasmid ratio CH3A (EW): CH3B (RVT) = 1:1, 1.5:1, 2:1) were prepared by dilution at a concentration of 1 µg/ml, and 45 µl of each of the resulting dilutions was added to 250 µl of inserts with Polyemer Feet, 100/PK (Agilent Technologies, USA), and injected into an autosampler vial to prepare a final sample to be analyzed. In addition, markers that enable the size of unknown protein samples to be distinguished were prepared under the same concentration conditions. The marker corresponding to 150 kDa is alcohol dehydrogenase and the marker corresponding to 66 kDa is BSA.

First, tertiary water in an amount corresponding to 2 to 3 times the column volume was flowed at 0.5 ml/min to remove an initial storage buffer (20% ethanol) from the column and at the same time, standardization was performed. After this process was completed, a physiological running buffer in an amount corresponding to 2 to 3 times the volume of 1X PBS (pH 7.4, 137 mM NaCl) was flowed at 0.75 ml/min to complete the standardization process before starting the analysis. The prepared analysis sample was placed in the device, the analysis was performed at the flow rate set to 0.75 ml/min, the injection amount was 30 µg (only 30 µl of the prepared 45 µl sample was injected), analysis was performed for 40 minutes, and post time was set for 10 minutes. When a determined injection amount of sample was injected by a sample injector, it flows into a tube through which the buffer flows and is injected into the column. When a buffer containing a sample passes through a column compressed with a porous resin, the time it takes for the protein in the sample to be eluted out of the column varies depending on the molecular weight, i.e., the size, of the protein, and the physical properties are analyzed through a chromatogram obtained by measuring the change in absorbance at 280 nm. At this time, the chromatogram may be interpreted based on the principle that as the size of the protein decreases, the amount of the protein that passes through the inside of the porous resin increases and the protein is eluted later, and as the size of the protein increases, the protein passes through the column through the outside of the porous resin and thus the protein is eluted faster.

The result of SEC analysis of wild-type Fc (γ1/4, WT), heterodimeric heavy chain constant region Fc (γ1/4, EW-RVT), and mono-IL21-Fc fusion protein (CH3A(EW) :CH3B(RVT)=1:1, 1.5:1, 2:1 when transformed) showed that the peak of mono-IL21-Fc fusion protein did not match the expected peak compared to the marker, but rather a peak corresponding to a smaller protein was observed. This meant that the biological properties of mono-IL21-Fc fusion protein were not good in 1X PBS, a storage buffer under physiological conditions, for reasons to be described later. Unstable biological properties mean that the mono-IL21-Fc fusion protein does not perform the desired function in the body and may have side effects and immunogenicity, which indicates that improvement of the biological properties of mono-IL21-Fc fusion protein was required.

### Example 4. Analysis of changes in SEC results of mono-IL21-Fc fusion protein depending on composition of analysis buffer

The results of SEC analysis performed in Example 3 showed that the properties of the mono-IL21-Fc fusion protein were poor. To determine the cause, the structure of human IL21 was analyzed, and the results are shown in FIG. 5. There were 22 amino acids with positive side chains on the surface of IL21. Among the amino acids with positive side chains, 10 arginines had the greatest effect. The isoelectric point of IL21 is 9.42, and IL21 is positively charged under pH 7.4 conditions. Based on this, it was assumed that the positive side chain on the surface of IL21 interacts nonspecifically with the column used for SEC analysis. Therefore, using high salt PBS (12 mM phosphate, 500 mM NaCl, 2.7 mM KCl, pH 7.4), which is a running buffer containing a high concentration of salt that creates an environment capable of offsetting the positive surface charge of IL21, SEC analysis was performed in the same manner as the SEC analysis method described in Example 4. The result showed that a peak having a level corresponding to the mono-IL21-Fc fusion protein was observed. The result is shown in FIG. 6B. As a control, the mono-IL21-Fc fusion protein was analyzed using 1X PBS, which provides a biological environment as a running buffer, and the result is shown in FIG. 6A. The result analysis showed that a peak corresponding to the protein of the mono-IL21-Fc fusion protein was observed, which demonstrates that human IL21 is strongly positively charged in a physiological condition buffer, and thus interacted nonspecifically with the column during SEC analysis and no peak was observed under the 1X PBS condition. In addition to SEC analysis, the positive surface charge of IL21 causes side effects due to nonspecific binding to cells and tissues other than the IL21 receptor when injected into a living body, and causes poor pharmacokinetics, thus making it difficult to function as a therapeutic agent. Therefore, in the following examples, a IL21 variant with a reduced positive surface charge was constructed.

### Example 5. Construction and expression/purification of IL21 variant of mono-IL21-Fc fusion protein

In order to reduce the surface positive charge of IL21, mono-IL21(NKQ)-Fc, mono-IL21(DDD)-Fc, mono-L21(NQNT)-Fc, and mono-IL21(DDD NQNT)-Fc were constructed as variants with substituted amino acids having positive side chains (sequence information is provided in Table 4). Specifically, first, in order to obtain information on the amino acids exposed on the surface of IL21, the structure was analyzed using the Chimera X program using the previously elucidated protein structure (PDB ID: 3TGX) as a sample. The structural analysis results showed that there were 22 amino acids with a positive surface charge, 10 of which were arginine, which have a high aqueous pKa (~13.8). In addition, when the structure was examined through a Chimera X program, an atypical region whose structure was not precisely identified could be observed. The mono-IL21-Fc fusion protein amino acid substitution variants were constructed by substituting amino acids in the atypical region or substituting amino acids in the region whose structure was identified.

The atypical region greatly contributes to the formation of unstable structures. IL21 has increased nonspecificity and increased possibility of forming oligomers, and worsens the physical properties due to the presence of three arginines (83R, 84R, 124R) in the atypical region. In particular, IL21 has nonspecific interactions with the SEC column, thus inducing results, as shown in FIG. 4. Therefore, a new clone was constructed by substituting arginines in the atypical region with uncharged polar amino acids or lysines with weaker positive charge than arginine with reference to the sequence of the central (rabbit, pig) (R83N, R84K, R124Q), which was referred to as "mono-IL21(NKQ)-Fc" (SEQ ID NO: 5).

A new clone was constructed by substituting arginine (R83, R84, R124) in the atypical region with aspartic acid, which has the strongest negative side chain among amino acids (R83D, R84D, R124D), which was referred to as "mono-IL21(DDD) -Fc" (SEQ ID NO: 6).

In addition, when the IL21 structure was analyzed using the Chimera X program (PDB ID: 3TGX), amino acids that are in the L21 receptor binding region or do not form significant bonds with surrounding residues and whose side chains are directed toward the surface were selected (K50, K54, K86, R88), and a new clone was constructed by substituting uncharged polar amino acids (K50N, K54Q, K86N, R88T) with reference with the ortholog sequence, which was referred to as "mono-IL21(NQNT)-Fc" (SEQ ID NO: 7).

A new clone was constructed by substituting amino acids (K50N, K54Q, R83D, R84D, K86N, R88T, R124D) including the substitution strategies of mono-IL21(DDD)-Fc and mono-IL21(NQNT) -Fc, which was referred to as "mono-IL21 (DDD NQNT) - Fc" (SEQ ID NO: 8).

The basis for the clones constructed in this example and the substitution strategy are shown in FIG. 7.

To construct mono-IL21(NKQ)-Fc, mono-IL21(DDD) -Fc, mono-IL21(NQNT)-Fc, and mono-IL21(DDD NQNT)-Fc clones, in-frame cloning was performed into the animal cell expression vector, pcDNA3.1(+) (Invitrogen, USA) using NotI/HindIII to form a signal sequence-IL21 (variants) mature form-Mutated Hinge-CH2-CH3A (IgG4, EW).

The mono-IL21-Fc fusion protein, mono-IL21(NKQ)-Fc, mono-IL21(DDD)-Fc, mono-IL21(NQNT)-Fc, and mono-IL21(DDD NQNT)-Fc were constructed by transiently transfecting HEK293-F (Invitrogen) cells with a mixture of polyethylenimine (PEI) (Polyscinece) and the expression vectors of IgG4 CH3A (EW) and IgG4 CH3B (RVT) fused with human IL21 wild-type and mutants (NKQ or DDD or NQNT or DDD NQNT) at a ratio of 2:1, and culturing the cells in a shake flask containing serum-free FreeStyle^{™} 293 expression medium (Invitrogen). The details of the method are as follows.

For transient transfection, the HEK293-F cells were seeded at a density of 1.0 × 10⁶ cells/ml in 90 ml of medium in a shaker flask (Corning) and incubated at 135 rpm in 8% CO₂. After 24 hours, to produce fusion proteins including each heterodimeric heavy chain constant region, the corresponding CH3A and CH3B plasmids were diluted in 4 ml FreeStyle^{®} 293 expression medium (Invitrogen) at a total of 125 µg (1.25 µg/ml), more specifically, at IgG4 CH3A (EW): IgG4 CH3B (RVT)=2:1=83.3 µg:41.7 µg, filtered, mixed with 6 ml of medium (3.75 µg/ml) in which 375 µg of PEI was diluted, and reacted at room temperature for 10 minutes. Then, the reacted mixed medium was added to the cells seeded in 90 ml in advance and cultured for 5 days at the shortest and 7 days at the longest. As a result, the proteins produced by the cells, that is, the fusion proteins containing a heterodimeric heavy chain constant region are secreted outside the cells by the cells and accumulated in the medium. Therefore, the proteins were purified using a protein A Sepharose column (GE healthcare) from the cell culture supernatant collected by centrifugation at 3,800 rpm for 25 minutes after cell culture. At this time, the purification was performed in accordance with the standard protocol provided by the protein A column company, and the eluted protein was concentrated after exchanging storage buffer (PBS, pH 7.4) using a Vivaspin 30,000 MWCO (Satorius) centrifugal concentrator. Absorbance of the purified protein was measured at a wavelength of 562 nm using a solution in the BCA protein assay kit (Thermo) and assayed in accordance with the drawn standard curve (Table 5). Experiments were independently performed in duplicate and the results are expressed as the standard error of the mean (mean ± SD). The result of the yield verification showed that the mono-IL21-Fc fusion protein variants had overall higher yield compared to the mono-IL21-Fc fusion protein. This indicates that there was no problem in the expression of the mono-IL21-Fc fusion protein amino acid substitution mutant constructed through the rationale.

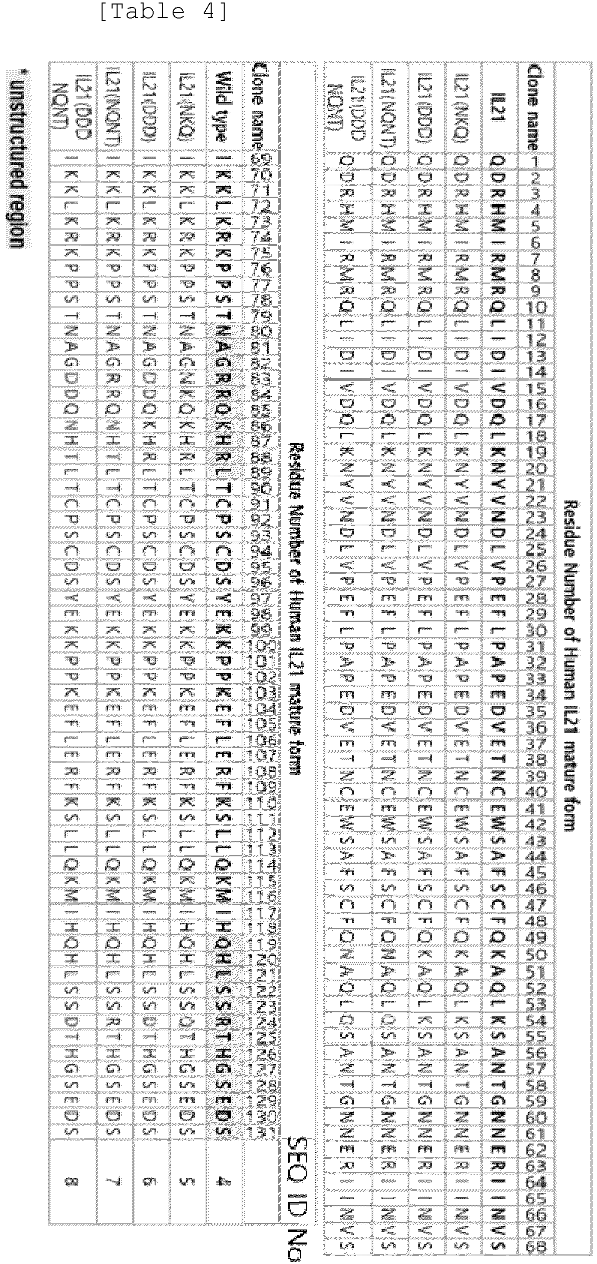

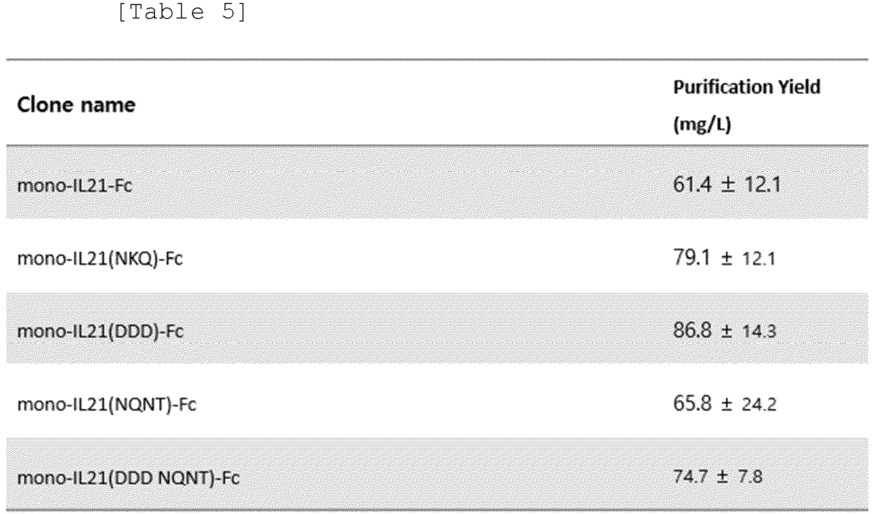

The purified mono-IL21-Fc fusion protein, mono-IL21(NKQ)-Fc, and mono-IL21(DDD)-Fc (5 µg) were analyzed through SDS-PAGE under 10% non-reducing and reducing conditions (FIG. 8). Under non-reducing conditions, mono-IL21-Fc fusion protein, mono-IL21(NKQ)-Fc, and mono-IL21(DDD)-Fc were observed at 71 kDa. Under reducing conditions, mono-IL21-Fc fusion protein, mono-IL21(NKQ)-Fc, and mono-IL21(DDD)-Fc, which include two plasmids including heterodimeric heavy chain constant region CH3A (EW) and heterodimeric heavy chain constant region CH3B (RVT) fused with human wild-type IL21 and variant (NKQ or DDD) had two bands at 43 and 28 kDa. The heterodimeric heavy chain constant regions CH3A and CH3B were each expected to have a protein size of about 25 kDa, and human IL21 was expected to have a protein size of 15 kDa, but bands larger than the expected protein size were observed under reducing and non-reducing conditions due to N-glycosylation in human IL21 and Fc. The SDS-PAGE results showed that there was no problem with the expression of each heterodimeric heavy chain constant region of the mono-IL21-Fc fusion protein amino acid substitution variations and the formation of the fusion protein using the same.

### Example 6. Evaluation of properties of mono-IL21-Fc fusion protein amino acid substitution variants

FIG. 8 is a chromatogram showing the mAU at 280 nm as a function of time (minutes) upon SEC (size exclusion chromatography) to analyze physical properties of the expressed mono-IL21 (NKQ)-Fc, mono-IL21(DDD)-Fc, and mono-IL21(NQNT)-Fc. The measurements were performed using an HPLC (Agilent Technologies, USA) instrument and a Superdex200 10/300 GL column (Cytiva, USA) according to the following specific protocol.

Tertiary water having undergone filtration and degassing, and 1X PBS (pH 7.4, 137 mM NaCl) (12 mM phosphate, 137 mM NaCl, 2.7 mM KCl, pH 7.4) were prepared. Mono-IL21-Fc fusion protein, mono-IL21(NKQ)-Fc, mono-IL21(DDD)-Fc, and mono-IL21(NQNT)-Fc were prepared by dilution at a concentration of 1 µg/ml, and 45 µl of each of the resulting dilutions was added to 250 µl of inserts with Polyemer Feet, 100/PK (Agilent Technologies, USA), and injected into an autosampler vial to prepare a final sample to be analyzed. In addition, markers that enable the size of unknown protein samples to be distinguished were prepared under the same concentration conditions. The marker corresponding to 150 kDa is alcohol dehydrogenase and the marker corresponding to 66 kDa is BSA.

First, tertiary water in an amount corresponding to 2 to 3 times the column volume was flowed at 0.5 ml/min to remove an initial storage buffer (20% ethanol) from the column and at the same time, standardization was performed. After this process was completed, a running buffer in an amount corresponding to 2 to 3 times the volume of 1X PBS (pH 7.4, 137 mM NaCl) was flowed at 0.75 ml/min to complete the standardization process before starting the analysis. The prepared analysis sample was placed in the device, the analysis was performed at a flow rate of 0.75 ml/min, the injection amount was 30 µg (only 30 µl of the prepared 45 µl sample was injected), analysis was performed for 40 minutes, and post time was set at 10 minutes.

The result of SEC analysis of mono-IL21-Fc fusion protein, mono-IL21(NKQ)-Fc, mono-IL21(DDD)-Fc, and mono-IL21(NQNT)-Fc showed that the peak corresponding to the expressed fusion protein, regarding the mono-IL21(NQNT)-Fc fusion protein variant was significantly increased compared to the mono-IL21-Fc fusion protein. This supported the hypothesis that the positive surface charge of human IL21 caused nonspecific interactions with the column during SEC analysis, which resulted in an unexpected peak during analysis. In addition, the mono-IL21-Fc fusion protein variant that alleviated this problem had improved biological properties, decreased nonspecific interactions when injected into the body, reduced drug side effects, and improved pharmacodynamics.

### Example 7. Construction of IL21 ortholog sequence graft mutant of mono-IL21-Fc fusion protein and expression/purification

Unlike the mono-IL21-Fc fusion protein variant constructed using amino acid substitution performed in Example 6 in order to further improve the biological properties of mono-IL21-Fc fusion protein, a mono-IL21-Fc fusion protein variant was developed using a new strategy. Specifically, first, the secondary structure formation patterns between the three cytokines, IL-2 (PDB ID: 1IAR) and IL-4 (PDB ID: 2B5I), which are cytokines of the same family as IL21, and IL21 were compared using PSIPRED, secondary structure prediction platform and multiple sequence alignment. The results of sequence analysis showed that IL21 had a shorter Helix C and a significantly longer loop structure connecting Helix C to Helix D than that of IL2 and IL4, and also had an atypical region between the loop structures. It was determined that these structural differences may make the structure of IL21 unstable. Therefore, the complementary sequences of the corresponding regions of IL2 and IL4 were compared with those of IL21 and a mono-IL21-Fc fusion protein ortholog sequence grafted mutant was constructed using the sequence of IL4, which has more hydrophilic residues than human IL2, which may cause structural instability when the sequence is grafted due to many hydrophobic amino acid residues. Thus, the sequence from K77 to R90, including the atypical region of IL21, was changed to the complementary human IL4 sequence and the ortholog IL4 sequence, to construct a mono-IL21-Fc fusion protein ortholog sequence transplant variant (sequence information is provided in Table 6). The variant using the human IL4 sequence was referred to as "mono-IL21(Helix 1)-Fc (SEQ ID NO: 9)" and the variant using the ortholog IL4 sequence was referred to as "mono-IL21(Helix 2)-Fc" (SEQ ID NO: 10).

The basis for the clones constructed in this example and the substitution strategy are shown in FIG. 9.

To construct mono-IL21 (Helix 1)-Fc, and mono-IL21(Helix 2)-Fc, in-frame cloning was performed into the animal cell expression vector, pcDNA3.1(+) (Invitrogen, USA) using NotI/HindIII to form signal sequence-IL21 (Helix 1) mature form-Mutated Hinge-CH2-CH3A (IgG4, EW), and signal sequence-IL21 (Helix 2) mature form-Mutated Hinge-CH2-CH3A (IgG4, EW).

Mono-IL21-Fc fusion protein, mono-IL21(Helix 1)-Fc, and mono-IL21(Helix 2)-Fc are constructed by transiently transfecting HEK293-F (Invitrogen) cells with a mixture of polyethylenimine (PEI) (Polyscinece) and expression vectors of IgG4 CH3A (EW) and IgG4 CH3B (RVT) fused with the expression vectors of wild type human IL21 or IL21 (Helix 1) or IL21 (Helix 2) at a ratio of 2:1, and culturing the result in a shake flask containing serum-free FreeStyle^{™} 293 expression medium (Invitrogen). The details of the method are as follows.

For transient transfection, HEK293-F cells were seeded at a density of 1.0 × 10⁶ cells/ml in 90 ml of medium in a shaker flask (Corning) and incubated at 135 rpm in 8% CO₂. After 24 hours, to produce fusion proteins including each heterodimeric heavy chain constant region, the corresponding CH3A and CH3B plasmids were diluted in 4 ml FreeStyle^{®} 293 expression medium (Invitrogen) at a total of 125 µg (1.25 µg/ml), more specifically, at CH3A (EW): CH3B (RVT)=2:1=83.3 µg:41.7 µg, filtered, mixed with 6 ml of medium (3.75 µg/ml) in which 375 µg of PEI was diluted, and reacted at room temperature for 10 minutes. Then, the reacted mixed medium was added to the 90 ml cells seeded in advance and cultured for 5 days at the shortest and 7 days at the longest. As a result, the proteins produced by the cells, that is, the fusion proteins containing a heterodimeric heavy chain constant region are secreted outside the cells by the cells and accumulated in the medium. Therefore, the proteins were purified using a protein A Sepharose column (GE healthcare) from the cell culture supernatant collected by centrifugation at 3800 rpm for 25 minutes after cell culture. At this time, the purification was performed in accordance with the standard protocol provided by the protein A column company, and the eluted protein was concentrated after exchanging storage buffer (PBS, pH 7.4) using a Vivaspin 30,000 MWCO (Satorius) centrifugal concentrator. Absorbance of the purified protein was measured at a wavelength of 562 nm using a solution in the BCA protein assay kit (Thermo) and assayed in accordance with the drawn standard curve (Table 7). Experiments were independently performed in triplicate and the results are expressed as the standard error of the mean (mean ± SD). The result of the yield verification showed that the yields of mono-IL21 (Helix 1)-Fc and mono-IL21 (Helix 2)-Fc were similar to that of the mono-IL21-Fc fusion protein.

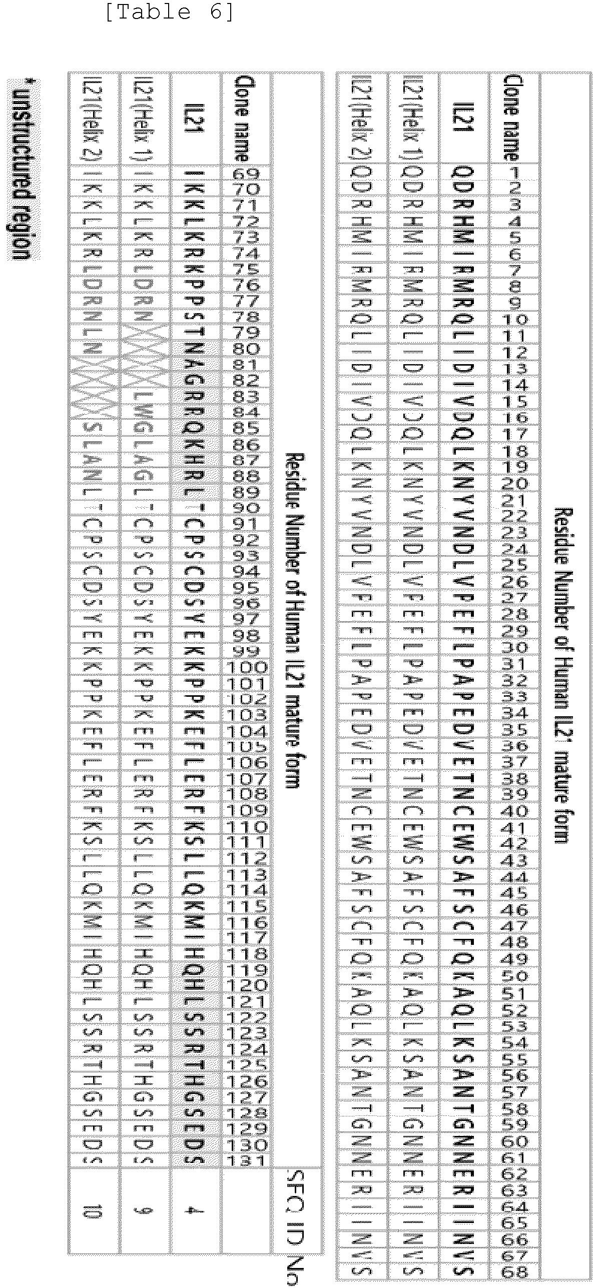

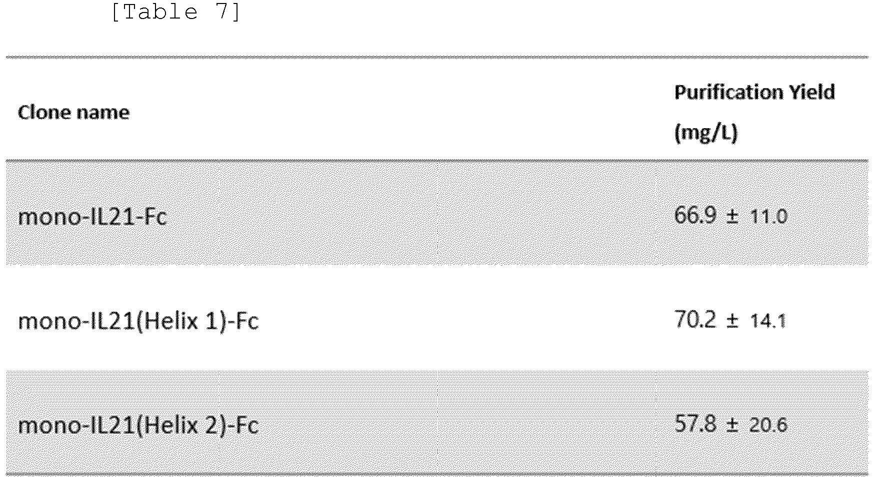

The purified mono-IL21-Fc fusion protein, mono-IL21(Helix 1)-Fc, and mono-IL21(Helix 2)-Fc (5 µg) were analyzed through SDS-PAGE under 10% non-reducing and reducing conditions (FIG. 11). Under non-reducing conditions, mono-IL21-Fc fusion protein, mono-IL21(Helix 1)-Fc, and mono-IL21(Helix 2)-Fc were observed at 71 kDa. Under reducing conditions, mono-IL21-Fc fusion protein which includes two plasmids including heterodimeric heavy chain constant region CH3A (EW) and heterodimeric heavy chain constant region CH3B (RVT) fused with human wild-type IL21 and IL21 (Helix 1) had two bands at 43 and 28 kDa. The heterodimeric heavy chain constant region CH3A (EW) fused to IL21 (Helix 2) was observed to have a band larger than 43 kDa. This was considered to result from the creation of another N-glycosylation region (N90-T92) when the IL4 sequence of the centromere was transplanted during the construction of IL21 (Helix 2). The result of SDS-PAGE, analysis showed that there was no problem with the expression of each heterodimeric heavy chain constant region and the formation of fusion proteins through the same.

Example 8. Evaluation of properties of IL21 ortholog amino acid substitution variants of mono-IL21-Fc fusion protein

FIG. 12 is a chromatogram showing the mAU at 280 nm upon SEC (size exclusion chromatography) to compare physical properties between the expressed IL21 ortholog amino acid substitution variants of mono-IL21-Fc fusion protein and the previously constructed mono-IL21-Fc fusion protein, mono-IL21(DDD) -Fc, mono-IL21(NQNT)-Fc, and mono-IL21(DDD NQNT)-Fc. The measurements were performed using an HPLC (Agilent Technologies, USA) instrument and a Superdex200 10/300 GL column (Cytiva, USA) according to the following specific protocol.

Tertiary water having undergone filtration and degassing, and 1X PBS (pH 7.4, 137 mM NaCl) were prepared. The constructed mono-IL21-Fc fusion protein, mono-IL21(DDD)-Fc, mono-IL21(NQNT)-Fc, mono-IL21(DDD NQNT)-Fc, mono-IL21(Helix 1)-Fc, and mono-IL21(Helix 2)-Fc were prepared by dilution at a concentration of 1 µg/ml, and 45 µl of each of the resulting dilutions were added to 250 µl of inserts with Polyemer Feet, 100/PK (Agilent Technologies, USA), and injected into an autosampler vial to prepare a final sample to be analyzed. In addition, standard samples that enable the size of unknown protein samples to be distinguished were prepared under the same concentration conditions. The standard sample corresponding to 150 kDa is alcohol dehydrogenase and the standard sample corresponding to 66 kDa is BSA.

First, tertiary water in an amount corresponding to 2 to 3 times the column volume was flowed at 0.5 ml/min to remove an initial storage buffer (20% ethanol) from the column and at the same time, standardization was performed. After this process was completed, a running buffer in an amount corresponding to 2 to 3 times the volume of 1X PBS (pH 7.4, 137 mM NaCl) was flowed at 0.75 ml/min to complete the standardization process before starting the analysis. The prepared analysis sample was placed in the device, the analysis was performed at a flow rate of 0.75 ml/min, the injection amount was 30 µg (only 30 µl of the prepared 45 µl sample was injected), analysis was performed for 40 minutes, and post time was set to 10 minutes.

The properties of the mono-IL21-Fc fusion protein ortholog sequence transplant variations were analyzed through size exclusion chromatography. As can be seen from FIG. 10, mono-IL21 (Helix 1)-Fc and mono-IL21 (Helix 2)-Fc had higher main peaks than the mono-IL21-Fc fusion protein. In particular, mono-IL21 (Helix 2)-Fc has a lower oligomer ratio and a higher main peak compared to the mono-IL21-Fc fusion protein. It is presumed that this is why the structural stability was further improved by reducing the number of arginines with positive side chain charges from 3 to 1 in the part where the sequence was changed and substituting the atypical region with a helix structure.

### Example 9. Evaluation of receptor binding ability of mono-IL21-Fc fusion protein

In order to evaluate the binding ability of the constructed mono-IL21-Fc fusion protein variants to the IL21 receptor, the receptor binding ability of mono-IL21-Fc fusion protein, mono-IL21(NKQ)-Fc, and mono-IL21(DDD)-Fc was evaluated using the Jurkat cell line, a CD4 positive human T cell line expressing the IL21 receptor. Specifically, cells were prepared at 2.0×10⁶ cells/sample, washed with washing buffer (PBS + 1% FBS), and then treated with mono-IL21-Fc fusion protein, mono-IL21(NKQ)-Fc and Mono-IL21(DDD)-Fc at 4°C for 1 hour at 200 nM and 50 nM. The result was washed again with washing buffer, treated with the secondary antibody, anti-human IgG4 FITC, at 4°C under dark conditions for 30 minutes. Then, the result was washed with washing buffer, and was analyzed using a flow cytometry instrument, FACScalibur (BD bioscience, USA).

The binding ability to the IL21 receptor was evaluated by labeling an experimental group that underwent the process of treating mono-IL21-Fc fusion protein and mono-IL21(variants)-Fc and a control group that did not undergo the process with a fluorescent substance, and measuring the intensity of the fluorescence signal. The results are shown in FIG. 13. The result showed that mono-IL21(variants) -Fc also had a fluorescence signal similar to the mono-IL21-Fc fusion protein, and that the intensity of the signal also increases as the concentration of the processed protein increases. This indicates that the receptor binding ability of mono-IL21(variants)-Fc is maintained and thus there is no problem with the biological activity.

### Example 10. Evaluation of biological activity of mono-IL21-Fc fusion protein variants

Human blood was collected with consent in accordance with the due process, and blood was carefully placed on the Ficoll layer at a ratio of 1:2 using Ficoll (Ficoll-paqueTM PLUS, GE healthcare), and centrifuged at 2,000 rpm for 30 minutes, and peripheral blood mononuclear cells (PBMCs) were separated from the buffy coat separated by the difference in density (specific gravity) (FIG. 14). In addition, the group was cultured after removing CD4 T cells and CD8 T cells using magnetic beads (Myltenyi Biotech) to enrich NK cells in PBMC separated by Ficoll (FIG. 15). Then, Jurkat cells and EBV-LCL cells irradiated with 100 Gy of gamma radiation were cultured in a ratio of 1:1 with peripheral blood mononuclear cells in medium supplemented with 10% FBS (Fetal Bovine Serum, HyClone), 1% penicillin-streptomycin (Gibco), and IL-2-Fc (monomer, 30 ng/ml) in RPMI 1640 (Corning). At this time, the cells were treated with 30 ng/ml of 7 types of mono Fc-IL21 fusion protein variants (Mono-IL21-Fc, Mono-IL21 (NKQ)-Fc, Mono-IL21 (DDD)-Fc, Mono-IL21 (NQNT)-Fc, Mono-IL21 (DDD NQNT)-Fc, Mono-IL21 (Helix 1)-Fc, Mono-IL21 (Helix 2)-Fc) once on Day 0. RPMI medium containing 30 ng/ml of IL-2-Fc (monomer) was added every 3 to 4 days, and the cell number was adjusted to 2.5 × 10⁵ cells/ml and transferred to a flask on Day 6 for culture. On day 6 and day 10, cells were fluorescently stained with CD3- (FITC) and CD56+ (APC), the percentage of NK cells was measured by flow cytometry, and the expansion ratio of NK cells was finally calculated. The result showed that, when PBMCs were used, NQNT and DDD-NQNT had a significantly higher yield than WT-IL21 (FIG. 14), and when PBMCs from which CD4 and CD8 T cells were removed were used, NQNT and helix-1 had a high yield (FIG. 15). It can be seen from this that the NK cell proliferation expansion ability was improved in the group treated with the mono Fc-IL21 fusion protein variant. It can be seen that, when the group was further treated with the mono Fc-IL21 fusion protein variant using the conventional treatment method during the *in vitro* expansion culture of NK cell therapeutic agents, a large amount of NK cells can be expanded compared to the conventional method.

### [Industrial applicability]

The expected limitation of the existing mono-IL21-Fc fusion protein disclosed herein is that, due to the high isoelectric point of wild-type IL21, the net charge is positive in the pH environment of the body, and a cationic patch is formed on the surface, which may non-specifically interact with various tissues and cells in addition to the IL21 receptor. Accordingly, when the mono-IL21-Fc fusion protein is injected into the body, it may have poor biological properties in the body, thus causing side effects such as excessive immune response through non-specific interactions with other cells and tissues, and resulting in poor pharmacodynamics. As a strategy to overcome this, the mono-IL21-Fc fusion protein or variant thereof, in which human IL21 or variant thereof is fused to the heterodimeric heavy chain constant region Fc (γ1/4, EW-RVT), maintains the structure of the conventional mono-IL21-Fc fusion protein, the binding ability to the IL21 receptor, and the biological activity including the proliferation ability and activation ability for T cells and NK cells, thereby improving the physical properties in the body, reducing side effects, and inducing the effect of increasing the half-life while maintaining the antitumor function.

The mono-IL21-Fc fusion protein or variant thereof according to the present invention is easily developed into a therapeutic drug based on high production yield, and can maintain the binding ability to the IL21 receptor of the conventional mono-IL21-Fc fusion protein, maintain the original biological function (increased T cell proliferation ability and toxicity ability, and increased NK cell proliferation ability and toxicity ability), and thus exhibit effective anticancer activity.

The mono-IL21-Fc fusion protein or variant thereof according to the present invention is useful as a pharmaceutical composition that can increase the proliferation and activation ability of T cells and NK cells used in immune cell therapy *in vitro* and *in vivo.*

The mono-IL21-Fc fusion protein or variant thereof according to the present invention is useful for basic experiments on immune cell culture to increase the proliferation and activation ability of T cells and NK cells used in immune cell therapy and for research on the development of cell therapies.

The construction strategy of the mono-IL21-Fc fusion protein or variant thereof according to the present invention provides a strategy for constructing a variant that is applicable to substances used as pharmaceutical compositions for the treatment and prevention of various diseases that are expected to have poor biological properties, side effects, and poor pharmacodynamics due to high positive charge in the body, in addition to IL21.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Sequence Listing Free Text]

An electronic file is attached.

## Claims

1. An interleukin 21 (IL21) variant in which one or more selected from the group consisting of amino acid sequences at positions 50, 54, 75 to 88 and 124 from an N-terminus in an interleukin 21 (IL21) including an amino acid sequence of SEQ ID NO. 4 are substituted or deleted.

2. The interleukin 21 (IL21) variant according to claim 1, wherein the interleukin-21 variant comprises one or more selected from the group consisting of the following amino acid substitutions and deletions in the amino acid sequence of SEQ ID NO: 4:
(1) K50N;
(2) K54Q;
(3) K75L;
(4) P76D;
(5) P77R;
(6) S78N;
(7) T79L;
(8) R83N, R83D, R83L;
(9) R84K, R84D, R84W;
(10) Q85G, Q85S;
(11) K86N, K86L;
(12) H87A;
(13) R88T, R88G, R88N;
(14) R124Q, R124D;
(15) deletion of amino acid at positions 79 to 82; and
(16) deletion of amino acid at positions 81 to 84.

3. The interleukin 21 (IL21) variant according to claim 1, wherein the interleukin-21 variant comprises one or more selected from the group consisting of the following amino acid substitutions and deletions in the amino acid sequence of SEQ ID NO: 4:
(1) R83N, R84K, R124Q;
(2) R83D, R84D, R124D;
(3) K50N, K54Q, K86N, R88T;
(4) K50N, K54Q, R83D, R84D, K86N, R88T, R124D;
(5) K75L, P76D, P77R, S78N, and amino acid deletions at positions 79 to 82, R83L, R84W, Q85G, K86L, H87A, R88G; and
(6) K75L, P76D, P77R, S78N, T79L, amino acid deletions at positions 81 to 84, and Q85S, K86L, H87A, R88N.

4. The interleukin 21 (IL21) variant according to claim 1, wherein the interleukin 21 variant comprises a sequence selected from the group consisting of sequences of SEQ ID NOS: 5 to 10.

5. A fusion protein comprising the interleukin 21 variant according to any one of claims 1 to 4.

6. The fusion protein according to claim 5, further comprising an antibody (immunoglobulin) heavy chain constant region (Fc) pair including a first Fc region and a second Fc region,
wherein the antibody (immunoglobulin) heavy chain constant region (Fc) pair has a mutation in a CH3 domain of the first Fc region or the second Fc region.

7. The fusion protein according to claim 6, wherein the interleukin 21 variant binds to an N-terminal of the first Fc region or the second Fc region.

8. The fusion protein according to claim 6, wherein the IL-21 or a variant thereof binds to the first Fc region or the second Fc region via a linker.

9. The fusion protein according to claim 8, wherein the linker comprises (GS)n, (GGS)n, (GSGGS)n or (GnS)m (wherein n and m are each 1 to 10).

10. The fusion protein according to claim 6, wherein each of the first Fc region and second Fc region is derived from an Fc region selected from the group consisting of human IgG1, IgG2, IgG3, IgG4, IgM, IgA, IgD and IgE.

11. The fusion protein according to claim 6, wherein the mutation of the CH3 domain of the first Fc region or the second Fc region comprises at least one selected from the group consisting of the following mutations, wherein a mutation position is determined in accordance with the EU index:
(1) substitution of amino acid at position K360 in the CH3 domain of the first Fc region with K360E;
(2) Substitution of amino acid at position K409 in the CH3 domain of the first Fc region with K409W;
(3) Substitution of amino acid at position E347 in the CH3 domain of the second Fc region with E347R; and
(4) Substitution of amino acid at position F405 in the CH3 domain of the second Fc region with F405T and substitution of amino acid at position D399 in the CH3 domain of the second Fc region with D399V.

12. The fusion protein according to claim 11, wherein the CH3 domain of the first Fc region or the second Fc region further comprises the following substituted amino acid, wherein a mutation position is determined in accordance with the EU index:
(i) cysteine (C) substituted at position Y349 in the CH3 domain of the first Fc region; or
(ii) cysteine (C) substituted at position S354 in the CH3 domain of the second Fc region.

13. The fusion protein according to claim 6, wherein the CH3 domain mutation of the first Fc region and the second Fc region comprises the following mutation, wherein a mutation position is determined in accordance with the EU index:
(1) substitution of amino acid at position K360 with K360E and substitution at position K409 with K409W in the CH3 domain of the first Fc region; and
(2) substitution of amino acid at position E347 with E347R, substitution at position F405 with F405T, and substitution at position D399 with D399V in the CH3 domain of the second Fc region.

14. The fusion protein according to claim 6, wherein the fusion protein comprises:
the interleukin 21 variant including a sequence selected from the group consisting of sequences of SEQ ID NOS: 5 to 10; and
the antibody (immunoglobulin) heavy chain constant region (Fc) pair including a first Fc region comprising a sequence of SEQ ID NO: 1, and a second Fc region comprising a sequence of SEQ ID NO: 2.

15. A nucleic acid encoding the interleukin 21 variant according to any one of claims 1 to 4.

16. An expression vector comprising the nucleic acid according to claim 14.

17. A transformed recombinant cell comprising the expression vector according to claim 15.

18. A method of producing an interleukin 21 variant comprising:
(a) culturing the cell according to claim 16; and
(b) harvesting the interleukin 21 variant from the cultured cell.

19. A nucleic acid encoding the fusion protein according to claim 5.

20. An expression vector comprising the nucleic acid according to claim 19.

21. A transformed recombinant cell comprising the expression vector according to claim 20.

22. A method of producing a fusion protein comprising:
(a) culturing the cell according to claim 21; and
(b) harvesting the fusion protein from the cultured cell.

23. A composition for preventing or treating cancer comprising the interleukin 21 variant according to any one of claims 1 to 4 or the fusion protein according to any one of claims 5 to 13 as an active ingredient.

24. A culture method comprising treating an immune cell with the interleukin 21 variant according to any one of claims 1 to 4 or the fusion protein according to any one of claims 5 to 13.

25. The culture method according to claim 24, wherein the immune cell comprises at least one selected from the group consisting of T cells, NK cells, cytokine-induced killer cells (CIKs), activated cytotoxic T lymphocytes (CTLs), macrophages, tumor-infiltrating lymphocytes (TILs), and dendritic cells.
